# EUROPEAN PATENT SPECIFICATION

(11) **EP 4 178 579 B1**
(45) Date of publication and mention of the grant of the patent: **12.02.2025**
(21) Application number: 21755360.1
(22) Date of filing: 08.07.2021
(51) Int. Cl.: A61K 31/4985, A61K 31/573, A61P 43/00, A61K 45/06

(54) **5-AMINO-2,3-DIHYDRO-1,4-PHTHALAZINEDIONE AS GLUCOCORTICOID-SPARING AGENT**
5-AMINO-2,3-DIHYDRO-1,4-PHTHALAZINDION ALS GLUCOCORTICOID-SPARENDES MITTEL
5-AMINO-2,3-DIHYDRO-1,4-PHTHALAZINEDIONE COMME AGENT D'ÉPARGNE GLUCOCORTICOÏDE

(30) Priority: 09.07.2020 EP 20000248
(43) Date of publication of application: 17.05.2023
(73) Proprietor: MetrioPharm AG, 8021 Zürich (CH)
(72) Inventor: BRYSCH, Wolfgang, 13505 Berlin (DE); VON WEGERER, Jörg, 13597 Berlin (DE); LUDESCHER, Beate, 13439 Berlin (DE); SCHUMANN, Sara, 14554 Seddiner See (DE); KAISER, Astrid, 12305 Berlin (DE); SCHULZ, Petra, 13158 Berlin (DE)
(74) Representative: Gruber, Daniel
(86) International application number: PCT/EP2021/000078
(87) International publication number: WO 2022/008093

(56) References cited:
- WO-A1-2017/202496
- US-A1- 2017 368 063
- US-A1- 2019 358 227

## Description

Any references in the description to methods of treatment refer to the compounds, pharmaceutical compositions and medicaments of the present invention for use in a method for treatment of the human (or animal) body by therapy.

Corticosteroids are a class of steroid hormones that are synthesized in the adrenal glands and can be further subdivided in two groups according to their respective dominant physiological and pharmacological action: glucocorticoids and mineralocorticoids.

Whilst mineralocorticoids regulate the salt and water metabolism, glucocorticoids have strong anti-inflammatory effects. They suppress inflammation and immunity and are further involved in the breakdown of macronutrients like fats, carbohydrates, and proteins. Thus, both the naturally occurring glucocorticoids, i.e. in particular cortisol (also referred to as hydrocortisone or 11beta,17alpha,21-trihydroxypregn-4-en-3,20-dione) and cortisone (also referred to as corticosterone or 17-hydroxy-11-dehydro-corticosterone), as well as man-made derivatives thereof (synthetic glucocorticoids) are widely used in medicine for the treatment of various medical conditions, in particular for the treatment of conditions that are related to the immune system such as inflammatory diseases and allergic reactions.

Glucocorticoids are, for example, used to treat rheumatoid arthritis, osteoarthritis, gouty arthritis, ulcerative colitis, multiple sclerosis, asthma, severe psoriasis, some tumors, cerebral edema, chemotherapy induced nausea and vomiting, to prevent transplant rejection, and any further indication that requires a fast and considerable anti-inflammatory and/or immunosuppressive effect.

Glucocorticoids are mainly provided as injectables, for oral use, as ointments, eye drops and for inhalation. Dosage and treatment regimens vary considerably depending on the specific type of glucocorticoid, species, age, indication, acuteness, desired pharmacokinetics, and application form. Due to common serious adverse reactions and long-term use effects as outlined below very strict treatment regimens must be followed when administering glucocorticoids. In most cases the treatment starts with a relatively high initial dose to gain a favorable initial response. Afterwards the dose is decreased in small steps to the lowest possible dose that maintains an adequate clinical response. Both initial and maintenance dose must be determined and controlled in regular intervals by a physician. Detailed overviews on different and indication-dependent dosage regimes of, e.g., dexamethasone are provided in publicly available online databases, such as Drugs.com.

Generally, it can be stated that glucocorticoids have a great positive impact on the course of many diseases and condition, particularly conditions related to the immune system. On the other hand, the administration of glucocorticoids to subjects in need thereof also comes along with a high number of serious disadvantages.

Adverse drug reactions include amongst others, edema, muscle weakness, mood swings, and a reduced effectiveness of antibiotics and vaccines. A more extensive list of adverse drug reactions is provided in the following.

Thereby it should be considered that long-term use of glucocorticoids frequently leads to such serious conditions and adverse drug reactions.

Independent of the adverse reactions and conditions already mentioned long-term use of glucocorticoids sooner or later inevitably leads to an atrophy of the adrenal glands (adrenal insufficiency) and thus the organism's inability to produce cortisol. Therefore, after a longer treatment with glucocorticoids the dose has to be reduced stepwise and must not be suddenly stopped due to the danger of a so-called adrenal (or Addisonian) crisis with symptoms of nausea, vomiting and shock.

There are also numerous drug-drug-interactions that have to be considered when using glucocorticoids. Some drugs (e.g., troleandomycin, erythromycin, clarithromycin) and also estrogens reduce the ability of the liver to metabolize glucocorticoids, thus increasing their plasma level and hence both desired effects and adverse drug reactions. Other drugs reduce the plasma levels of glucocorticoids either by increasing its breakdown in the liver (e.g., phenobarbital, ephedrine, phenytoin, rifampicin) or by decreasing the absorption of orally taken glucocorticoids (e.g., cholestyramine). The effect of some other drugs (e.g., diuretics, amphotericin B, warfarin) is increased when glucocorticoids are given contemporarily, potentially leading to life-threatening situations.

Due to all these risks, in food of animal origin predefined maximum residue limits and waiting periods must be observed, some specific products may not be allowed at all. Thus, the veterinary application of glucocorticoids in livestock is additionally limited by administrative regulations.

### TASK

Due to these numerous drawbacks current treatment regimens with glucocorticoids are often regarded as unsatisfactory. There is a medical need for new agents and/or treatment regimens which allow to prevent and/or reduce unwanted effects of a glucocorticoid therapy, such as adverse drug reactions, drug-drug interactions, long-term effects, and habituation, while maintaining its therapeutic effect in a subject in need thereof.

Therefore, it is the task of the present invention to provide a glucocorticoid-sparing agent, such as a pharmaceutical drug, that allows to reduce the required dosages of glucocorticoids without reducing their desired effectiveness.

It is also a task of the present invention to provide an agent, such as a pharmaceutical drug, that allows to avoid and/or reduce adverse reactions of glucocorticoids as well as conditions related to long-term use of glucocorticoids.

Most preferable are thus combinations of glucocorticoids and at least one further agent acting synergistically in terms of efficacy whereas at the same time frequency, severity and seriousness of glucocorticoid related adverse reactions and conditions related to the long-term use of glucocorticoids are reduced.

### BRIEF SUMMARY OF THE INVENTION

Surprisingly, it was found that the administration of 5-amino-2,3-dihydro-1,4-phthalazinedione or one of its pharmaceutically acceptable salts in addition to a glucocorticoid therapy can reduce both the dosage of the glucocorticoid treatment without reducing its effectiveness, as well as decrease unwanted effects associated to glucocorticoids, as will be specified in detail in the Examples.

Particularly, the administration of 5-amino-2,3-dihydro-1,4-phthalazinedione sodium salt in addition to a glucocorticoid therapy allows to reduce the dosage of the glucocorticoid, as it was found that 5-amino-2,3-dihydro-1,4-phthalazinedione sodium salt and the glucocorticoid dexamethasone act in a supraadditive manner when administered concomitantly (see Example 1). Particularly, the administration of 5-amino-2,3-dihydro-1,4-phthalazinedione sodium salt in addition to a glucocorticoid therapy allows to decrease unwanted effects associated to glucocorticoids, as furthermore, it could be shown, that the concomitant administration of 5-amino-2,3-dihydro-1,4-phthalazinedione sodium salt and dexamethasone reduces unwanted effects related to dexamethasone (see Example 2).

The addition of 5-amino-2,3-dihydro-1,4-phthalazinedione or one of its pharmaceutically acceptable salts to an already ongoing glucocorticoid therapy can also help to avoid unwanted glucocorticoid effects and can further reduce those unwanted glucocorticoid effects that already have emerged.

In particular, the administration of 5-amino-2,3-dihydro-1,4-phthalazinedione sodium salt in addition to a glucocorticoid therapy can avoid and/or reduce unwanted glucocorticoid effects.

These unwanted effects comprise, without being limiting, hypertonia, weight gain and obesity (particularly truncal obesity, including the so called "buffalo hump"), edema, puffiness of the face (moon face), potassium loss, muscle weakness, headache, facial hair growth (in women), thinning of the skin, easy bruising and slow wound healing, glaucoma, cataracts, stomach and duodenum ulcers, irregular menstrual cycle, steroid induced diabetes, loss of control in existing diabetes, osteoporosis (resulting in bone fractures), adrenal joint necrosis (in particular of the hip or the knee joint), psychiatric disturbances (e.g. depression, euphoria, insomnia, mood swings, personality changes), psychotic behavior, growth retardation in children, convulsions, an increased rate of infections, exacerbation of opportunistic infections (such as tuberculosis, herpes zoster and *Pneumocystis* pneumonia), reduced effectiveness of antibiotics and vaccines, and in particular Cushing's syndrome, wherein the latter represents a collection of signs and symptoms that occur due to prolonged exposure to cortisol including for example high blood pressure, abdominal obesity, striae, "moon face", corticosteroid-induced lipodystrophy (e.g. buffalo hump), weak muscles, weak bones, fragile skin, acne, facial hair growth (in women) and irregular menstruation. Providing the possibility to reduce the dosage of glucocorticoid therapies by adding 5-amino-2,3-dihydro-1,4-phthalazinedione or one of its pharmaceutically acceptable salts to said therapies also helps to avoid and/or reduce unwanted effects associated to glucocorticoid removal. Particularly, the administration of 5-amino-2,3-dihydro-1,4-phthalazinedione sodium salt in addition to a glucocorticoid therapy allows to decrease unwanted effects associated to glucocorticoid removal such as adrenal insufficiency or even an acute Addisonian crisis including symptoms like nausea, vomiting and shock.

In case of such an adrenal insufficiency, corticosteroids with mineralocorticoid potency must be administered parenterally in addition to the glucocorticoids with anti-inflammatory potency to maintain the regulation of salt and water metabolism. Hence, 5-amino-2,3-dihydro-1,4-phthalazinedione or one of its pharmaceutically acceptable salts can be regarded as sparing agent for all corticosteroid drugs, particularly for glucocorticoids with additional mineralocorticoid potency.

5-amino-2,3-dihydro-1,4-phthalazinedione belongs to the pharmaceutical class of the phthalazinediones. Compounds of this class are known for their beneficial anti-inflammatory action. 5-amino-2,3-dihydro-1,4-phthalazinedione is also known under the name luminol. Luminol became known for its chemiluminescent properties. It is widely applied in diagnostic assays as a detection means and in forensic medicine, for example for tracing blood spots. In medicine, 5-amino-2,3-dihydro-1,4-phthalazinedione is developed in the form of a sodium salt suitable for use in the prophylaxis and treatment of a broad range of acute and chronic inflammatory disorders, including a.o. acute infections of bacterial and viral origin, particularly of the intestinal tract, hepatitis B and C, gastroenteritis, inflammations such as prostatitis, endometriosis, throat inflammation, bronchial asthma, pneumonia, periodontitis, pyelonephritis and autoimmune diseases such as Crohn's disease, ulcerative colitis, lupus erythematosus and scleroderma. Further, there is still a long list of indications in scientific and patent literature in the treatment of which 5-amino-2,3-dihydro-1,4-phthalazinedione was allegedly tested or a beneficial use was suggested (cf. WO 2004/041169; WO 2007/018546; WO 2012/127441 a.o.).

Some publications mention that 5-amino-2,3-dihydro-1,4-phthalazinedione or one of its pharmaceutically acceptable salts can be administered in combination with glucocorticoids (cf. WO 2017/202496; US 2019/358227; US 2017/368063). A use of 5-amino-2,3-dihydro-1,4-phthalazinedione or one of its pharmaceutically acceptable salts as a glucocorticoid-sparing agent in conditions treated with glucocorticoids thereby allowing a clear reduction of the glucocorticoid dose required, however, hasn't been described yet.

The present invention refers to 5-amino-2,3-dihydro-1,4-phthalazinedione or one of its pharmaceutically acceptable salts for use as glucocorticoid-sparing agent in the prophylaxis and/or treatment of conditions usually treated with glucocorticoids. In particular, the present invention refers to 5-amino-2,3-dihydro-1,4-phthalazinedione sodium salt for such a use.

While most conventional immunomodulatory drugs show serious adverse reactions, or are at least problematic in long-term treatment, 5-amino-2,3-dihydro-1,4-phthalazinedione or one of its pharmaceutically acceptable salts is well tolerated and has a high safety margin in respect to administered dosages (cf. Schumann et al., Int. J. Mol. Sci. 2020, 21(22), 8803). On the contrary, herein it was found that 5-amino-2,3-dihydro-1,4-phthalazinedione or one of its pharmaceutically acceptable salts avoids and/or reduces unwanted effects of glucocorticoids such as adverse reactions and/or conditions related to the long-term use of glucocorticoids.

Such a use of 5-amino-2,3-dihydro-1,4-phthalazinedione or one of its pharmaceutically acceptable salts in conditions treated with glucocorticoids to avoid and / or reduce unwanted effects related to the glucocorticoid therapy hasn't been described yet, either.

The present invention also refers to 5-amino-2,3-dihydro-1,4-phthalazinedione or one of its pharmaceutically acceptable salts for use as an agent to reduce or avoid unwanted glucocorticoid effects in conditions treated with glucocorticoids.

Further, the present invention refers to 5-amino-2,3-dihydro-1,4-phthalazinedione or one of its pharmaceutically acceptable salts for use as glucocorticoid-sparing agent in the prophylaxis and/or treatment of conditions usually treated with glucocorticoids further characterized by the use as an agent to reduce or avoid unwanted glucocorticoid effects. In particular the present invention refers to 5-amino-2,3-dihydro-1,4-phthalazinedione sodium salt for such uses.

Herein disclosed are thus the use of 5-amino-2,3-dihydro-1,4-phthalazinedione or one of its pharmaceutically acceptable salts, in particular of 5-amino-2,3-dihydro-1,4-phthalazinedione sodium salt, as a glucocorticoid-sparing agent and as an agent to reduce unwanted glucocorticoid effects.

Further disclosed are pharmaceutical combinations comprising 5-amino-2,3-dihydro-1,4-phthalazinedione or one of its pharmaceutically acceptable salts, in particular 5-amino-2,3-dihydro-1,4-phthalazinedionesodium sodium salt, as well as suitable application and treatment forms, application methods and treatment regimens, and pharmaceutically acceptable dosage forms - including pharmaceutical compositions comprising both a glucocorticoid and 5-amino-2,3-dihydro-1,4-phthalazinedione or one of its pharmaceutically acceptable salts, in particular 5-amino-2,3-dihydro-1,4-phthalazinedionesodium salt.

Methods of treatment are also disclosed.

### DETAILED DESCRIPTION

The invention as laid out in the brief summary section will be supported by detailed definitions and listings as provided in the following.

To avoid ambiguities in the art, the following terms of interaction among drugs are defined. They are used in this sense throughout the disclosure. If at least two substances (e.g., pharmaceutical agents) are administered concomitantly and at least one of these substances affects the activity of the other at least one substance a so-called drug-drug interaction is given. If said interaction leads to an exaggerated or increased effect of the drug substance the effect is synergistic. The respective equation can be displayed as (A+B) > A or (A+B) > B, wherein A and B are the percentual or fractional effects (i.e., a value between 0 and 1) seen when the respective substances are administered alone and (A+B) is the percentual or fractional effect seen after combinatory application, respectively. A final effect equal to the sum of the effects seen in the single drug applications is an additive synergistic effect. If the final effect is greater than said expected effect the term supraadditivity is used. Smaller effects are subadditive. If, on the other hand the drug-drug interaction leads to a decreased effect of the drug substance the effect is not synergistic at all, but antagonistic.

Hence, in the scope of this disclosure the terms "antagonism" and "antagonistic" are used for any decreased drug effect induced by drug-drug-interaction, whilst the terms "synergy" and "synergistic" are used for any increased drug effect induced by drug-drug-interaction. To describe and determine the grade of said synergy the terms "subadditive", "additive" and "supraadditive" as well as the respective nouns are used. Hence, the term supraadditivity is used to describe an effect of two or more combined agents that is greater than the expected additive effects of the single agents.

The use of synergistic acting drug combinations can both help to increase the therapeutic efficacy and the potency, wherein the latter primarily helps to reduce off-target toxicity.

The identification of drug-drug-interactions depends on the "no-interaction" null-hypotheses, which is based on the observed drug response and not on any model of mechanism. Hence, for two different drugs, their grade of additivity is based on a point of reference for the readout wherein the point of reference depends on the mathematical model chosen. Various methods exist to identify such interactions and to calculate the expected additive effect of two substances and thus to determine if the actual synergistic effect observed is subadditive, additive or supraadditive. Different methods are recommended in the art. The most common methods are outlined below. Basic methods like building simple arithmetic sums or the fractional product method provide an easy way to gain first insights if a specific combination has supraadditive potential.

The simple arithmetic sum method for additivity is based on the equation A + B = (A+B), wherein A and B again are the percentual or fractional effects seen when the respective substances are administered alone and (A+B) is the percentual or fractional effect seen after combinatory application of said substances using the same doses as in the single applications. If A + B > (A+B) supraadditivity is given. An obviously striking disadvantage of this method is that results wherein A + B ≥ 100% cannot be analyzed for supraadditivity. Hence, this method is primarily used to interpret combinations of suboptimal doses.

The fractional product method is based on the equation 1 - (1-A)*(1-B) = (A+B). Here, A and B are the fractual effects seen when the respective substances are administered alone and (A+B) is the fractual effect seen after combinatory application of said substances using the same doses as in the single applications. If 1 - (1-A)*(1-B) > (A+B), supraadditivity is given.

More sophisticated methods include the use of a so-called isobologram, a graph constructed on a coordinate system defined by individual drug doses showing a "line of additivity" that allows to distinguish between subadditive, additive (along the line) and supraadditive effects. The "line of additivity" connects those single drug doses which display the same effect (e.g., 50% inhibition of a specific marker). All possible dose combinations along this line are expected to show the same effectiveness. Dose combinations located within the triangle built by coordinates and the line of additivity, i.e., nearer to the arbitrary point, displaying the same effect are considered as supraadditive. Dose combinations located outside the triangle are considered as subadditive.

Respective figures can be mapped using specific software as e.g., CompuSyn (Chou TC and Martin N. ComboSyn, Inc. Paramus, NJ 2007 [www.combosyn.com]).

Also, further specific indicator values as for example the Combination Index (CI, equation of Chou-Talalay 1984) and the Dose-Reduction Index (DRI, equation of Chou 1984) can be calculated easily using the aforementioned specific software. Both these values allow to determine if drug substances act supraadditive synergistically when administered contemporarily. The CI is based on the principles of mass action law and is applicable for any kind of drug combination regardless of mechanism of action, dynamic order or the quantity units used for each drug in the combination. The CI value defines synergism as supraadditive if CI<1 and as additive if CI =1. If CI > 1 the effect is either subadditive or antagonistic. Hence, a CI of 1 also refers to the "line of additivity" In the classic isobologram as mentioned above. In a simplified approach it could be calculated as follows: CI = A(t)/A(x) + B(t)/B(x), wherein A(t) respectively B(t) is the dose of drug A respectively B alone that inhibits x %, whereas A(x) and B(x) stand for the portion of the respective drug in the combination that also inhibits x %.

The DRI is a measure of how many folds the dose of each drug in a synergistic combination may be reduced at a given effect level compared with the doses of each drug alone. Therefore, DRI=1 indicates additivity, whereas DRI>1 and <1 indicate supraadditivity and subadditivity (or antagonism), respectively.

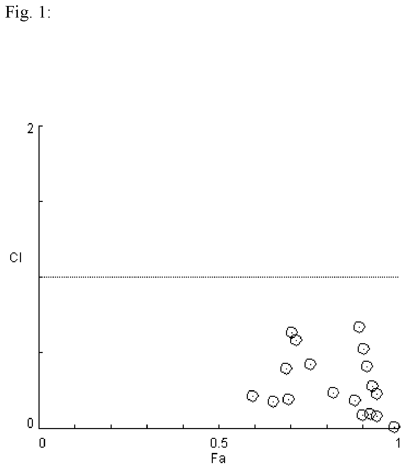

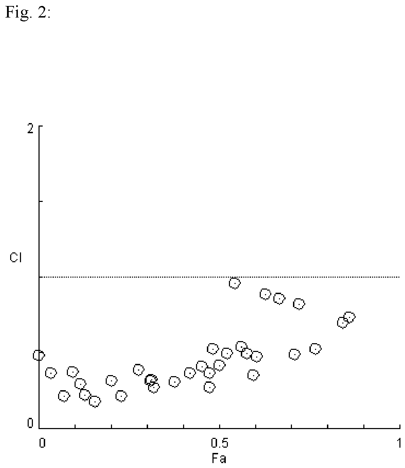

For displaying purposes, the isobologram, i.e., the equi-effective curve at various concentrations or doses of two drugs as mentioned above, is a dose-oriented figure approach based on a special case of the CI equation. A more convenient figure approach, however, is the effect oriented so-called FaCI plot, displaying the Combination Index (CI) against the fractual effect (Fa), preferably for a specific dose combination (see figures 1 and 2).

Some of the most common systemically applied corticosteroids are cortisone, hydrocortisone, prednisone, prednisolone, triamcinolone, methylprednisolone, betamethasone, dexamethasone, and fludrocortisone. They differ in their relative anti-inflammatory (glucocorticoid) and mineralocorticoid potency compared to the naturally occurring cortisol and in the duration of effects which are not accurately reflected by plasma half-lives. Within the corticosteroids listed above fludrocortisone is worth mentioning as mineralocorticoid which has the highest mineralocorticoid potency combined with an intermediate effect duration of approximately 24 hours, whereas all other examples are glucocorticoids with betamethasone and dexamethasone having the highest anti-inflammatory potencies combined with a long-term effect duration of 2 to 3 days.

In the scope of the present disclosure the term "corticosteroids" comprises any kind of naturally occurring or synthetic corticosteroids that are administered to human beings or animals to treat or prevent any kind of condition or disease. The term "glucocorticoids" comprises any "corticosteroids" displaying a considerable anti-inflammatory potency and affinity to the glucocorticoid receptor. Further the term "glucocorticoid" comprises any pharmaceutically acceptable forms of glucocorticoids such as esters, and salts.

In the sense of the present patent application if no other meaning is clearly specified the terms "condition" and "disease" refer to any type of condition, disease or disorder that might be prevented and / or treated by the administration of glucocorticoids.

Preferably the present invention relates to corticosteroids with a predominantly anti-inflammatory potential, i.e., glucocorticoids, including but not limited to flumethasone, triamcinolone acetonide, betamethasone, deflazacort, dexamethasone, beclomethasone, betamethasone valerate, betamethasone dipropionate, budesonide, beclomethasone dipropionate, isoflupredone, fluocinonide, fluocinolone, prednisone, prednisolone, methylprednisolone, halcinonide, desonide, deltasone, triamcinolone, triamcinolone acetonide, tixocortol pivalate, mometasone, amcinonide, fluocortolone, halometasone, alclometasone dipropionate, prednicarbate, clobetasone-17-butyrate, clobetasol-17-propionate, fluocortolone caproate, fluocortolone pivalate, fluprednidene acetate, ciclesonide, flunisolide, fluticasone furoate, fluticasone propionate, cortisol, hydrocortisone, hydrocortisone acetate, hydrocortisone-17-valerate, hydrocortisone-17-butyrate, hydrocortisone-17-aceponate, hydrocortisone-17-buteprate, cortisone and cortisone acetate.

The present invention most preferably relates to glucocorticoids selected from the group comprising cortisone, hydrocortisone, prednisone, prednisolone, triamcinolone, methylprednisolone, betamethasone, and dexamethasone.

The different substances are from a practical point of view used for different indications as they in their glucocorticoid potency in comparison to cortisol. Whilst cortisone for example is less potent than cortisol, the potency of prednisone is four times the potency of cortisol; prednisolone and methylprednisolone exhibit a five-fold potency; the potency of dexamethasone is even about 30 times the potency of cortisol.

A great variety of glucocorticoid formulations is available for oral, parenteral, and various topical uses, such as dermal, transdermal, ocular, and intraarticular. The present invention relates to any suitable glucocorticoid formulation. Preferably the present invention relates to formulations comprising at least one glucocorticoid for systemic or topical administration. Most preferably the present invention relates to systemically or topically administered glucocorticoids, wherein the administration mode is selected from a group comprising intravenous, oral, sublingual, rectal, topical and dermal administration.

As mentioned earlier, the present invention relates to 5-amino-2,3-dihydro-1,4-phthalazinedione as a corticoid sparing agent.

To ensure a better solubility and bioavailability pharmaceutically acceptable salts of 5-amino-2,3-dihydro-1,4-phthalazinedione may be used. Sodium, potassium, and lithium salts have been described for therapeutic applications (cf. WO 2010/082858). Thus, the present invention refers to pharmaceutically acceptable salts of 5-amino-2,3-dihydro-1,4-phthalazinedione selected from a group comprising sodium, potassium and lithium salts of 5-amino-2,3-dihydro-1,4-phthalazinedione. In particular, the present invention refers to 5-amino-2,3-dihydro-1,4-phthalazinedione sodium salt.

Crystal structures for lithium, sodium, potassium, rubidium, and cesium salts were described in Guzel et al. (2013) Journal of Coordination Chemistry 66, 3722-3739. Thus, the present patent application refers also to the use of crystalline forms of all pharmaceutically acceptable salts of 5-amino-2,3-dihydro-1,4-phthalazinedione.

5-amino-2,3-dihydro-1,4-phthalazinedione is often used as a hydrate, for example as sodium salt dihydrate. Thus, the present patent application refers also to the use of all hydrates and other solvates of 5-amino-2,3-dihydro-1,4-phthalazinedione and its pharmaceutically acceptable salts. 5-amino-2,3-dihydro-1,4-phthalazinedione, its derivatives or pharmaceutically acceptable salts may build complexes with suitable ligands. Thus, the present patent application refers also to such complexes.

To ensure a reproducible and standardized active pharmaceutical ingredient (API) production and to provide improved stability features of an active agent anhydrous formulations are often preferred. Anhydrate forms of 5-amino-2,3-dihydro-1,4-phthalazinedione sodium salt have been described as crystalline polymorphs in WO 2011/107295 (Form I, Form II) and WO 2016/096143 (Form III). These crystalline polymorphs are virtually free from phase impurities and were characterized by means of X-ray powder diffraction. This method yields a set of characteristic d-values indicating interplanar spacings and of the corresponding 2-theta (2θ) angles under which Bragg reflections occur. Additionally, the relative intensities (upon normalization to the respectively highest peak as 100%) of the reflections are indicated therein. This yields a unique and unambiguous fingerprint of the respective polymorphs.

For Form I the following values were determined: d values: 13.5; 6.9; 5.2; 4.6; 3.9; 3.5; 3.4; 3.3; 3.1; 3.0 and/or 2-theta values: 6.5; 12.7; 16.9; 19.3; 22.8; 25.8; 26.6; 27.2; 28.7; 30.3.

Form II is characterized by the following values: d values: 12.9; 7.9; 7.1; 6.5; 5.3; 4.0; 3.7; 3.6; 3.3; 3.2 and/or 2-theta values: 6.8; 11.2; 12.5; 13.7; 16.7; 22.4; 24.3; 24.9; 27.2; 27.8.

Form III yielded the following values: d values: 13.131; 7.987; 7.186; 6.566; 6.512; 5.372; 3.994; 3.662; 3.406; 3.288; 3.283; 3.222; 3.215; 3.127; 2.889 and/or 2-theta values: 6.73; 11.07; 12.31; 13.48; 13.59; 16.49; 22.24; 24.29; 26.14; 27.10; 27.14; 27.67; 27.72; 28.52; 30.93.

Thus, the present patent application refers also to the use of all crystalline forms and polymorphs thereof of 5-amino-2,3-dihydro-1,4-phthalazinedione and its pharmaceutically acceptable salts.

Similar therapeutic effects are known for a variety of phthalazinediones, respectively of derivatives of 5-amino-2,3-dihydro-1,4-phthalazinedione and its pharmaceutically acceptable salts. An example is 6-amino-2,3-dihydrophthalazine-1,4-dione (isoluminol). An overview of suitable phthalazinediones is given in WO 2007/018546. It is reasonable to assume that these compounds show comparable effects when being used for the therapeutic applications according to the invention.

Tautomerism relates to a rapid intraconversion of organic compounds in which a hydrogen atom or proton formally migrates inside the compound. This is accompanied by a switch of a single bond and adjacent double bond. The single forms are called tautomers. For example, keto-enol tautomerism occurs in 5-amino-2,3-dihydro-1,4-phthalazinedione. Thus, the present patent application refers also to the use of all tautomers of 5-amino-2,3-dihydro-1,4-phthalazinedione and its pharmaceutically acceptable salts.

As used throughout the present application the term "5-amino-2,3-dihydro-1,4-phthalazinedione" shall encompass all the aforementioned molecular variants of 5-amino-2,3-dihydro-1,4-phthalazinedione and its pharmaceutically acceptable salts, unless otherwise stated; particularly this term includes complexes, as well as solvates, hydrates, crystalline polymorphs, and tautomers. In the scope of the present application the term "combination" or "pharmaceutical combination" refers to the administration of at least two different active substances within the same treatment scheme for a specified indication. The term "composition" or "pharmaceutical composition" refers to a single pharmaceutical formulation including at least one active ingredient. Hence, the at least two active substances within a "pharmaceutical combination" can either be administered separately or within the same single "composition".

Thus, the present patent application refers to a pharmaceutical combination comprising at least one 5-amino-2,3-dihydro-1,4-phthalazinedione and at least one glucocorticoid for use in the prophylaxis and/or treatment of conditions or diseases usually treated with glucocorticoids, wherein the 5-amino-2,3-dihydro-1,4-phthalazinedione is either administered as add-on to at least one glucocorticoid or within the same pharmaceutical composition with the at least one glucocorticoid.

Preferably the present patent application refers to a pharmaceutical combination comprising at least one 5-amino-2,3-dihydro-1,4-phthalazinedione for use in the prophylaxis and/or treatment of conditions or diseases usually treated with glucocorticoids, characterized by lower doses of glucocorticoids, compared to the respective treatment with glucocorticoids alone.

The present patent application also refers to a pharmaceutical combination comprising at least one 5-amino-2,3-dihydro-1,4-phthalazinedione and at least one glucocorticoid for use in the prophylaxis and/or treatment of unwanted glucocorticoid effects, wherein the 5-amino-2,3-dihydro-1,4-phthalazinedione is either administered as add-on to at least one glucocorticoid or within the same pharmaceutical composition with the at least one glucocorticoid.

Herein the terms "medicine" and "medical" comprise both human and veterinary medicine.

According to the application and if not stated otherwise the terms "agent", "pharmaceutical agent", "compound", "active substance", or "active pharmaceutical ingredient" (API) refer to the substances or the compounds of 5-amino-2,3-dihydro-1,4-phthalazinedione and/or glucocorticoids as defined above.

The terms "composition" or "pharmaceutical composition" comprise at least one active substance in any pharmacologically acceptable defined dosage and dosage form together with at least one pharmaceutically acceptable excipient, as well as all agents that are generated from the aforementioned ingredients directly or indirectly as a combination, accumulation, complex or crystal, or as a consequence of other reactions or interactions, as well as optionally at least one further pharmaceutical drug.

The term "excipient" is used in this application to describe any component of a pharmaceutical composition apart of the active substance. The selection of suitable excipients depends on a variety of factors, such as the dosage form, the dosage, the desired solubility and stability of the composition.

The terms "effect", or "action" in regard to the active substance respectively the combination of the invention refers to causally occurring consequences in the organism to which said substance or combination has been administered before. In case of causally occurring beneficial consequences to the organism the terms "therapeutic effect", "therapeutic action" or "efficacy" are used. The term "effectiveness" refers to the desired degree of therapeutic effect.

The terms "unwanted glucocorticoid effects" or "unwanted effects related to glucocorticoids" comprise any unwanted effects associated to the treatment with glucocorticoids, such as adverse reactions, long-term side effects, habituation and also effects associated to the sudden removal of glucocorticoids.

According to the invention the terms "effective amount" and "therapeutically effective amount" refer to an amount of the substance of the invention that is sufficiently large to cause a desired beneficial effect in a person in need of such a prophylaxis or treatment.

The term "synergy" or "synergistic effect" refers to an effect resulting from the combinatory application of at least two substances showing comparable effects wherein the therapeutic effect of any of said substances is strengthened when the at least one further substance is added.

The terms "subadditive", "additive" and "supraadditive" refer to the grade of synergistic effect of substances administered in combination compared to the same substances administered as single agents.

The term "antagonism" or "antagonistic effect" refers to an effect resulting from the combinatory application of at least two substances showing comparable effects wherein the therapeutic effect of any of said substances is weakened when the at least one further substance is added.

In the scope of the present invention the terms "concomitant administration" or "administrated concomitantly" refers to the administration of at least two pharmaceutical agents within a certain timeframe, wherein the latter depends on the interval of the administration of the agent which is given at a lower frequency. For example, if the agent with the longer administration interval is given daily the second agent has to be administered on the same day for concomitant use. In case of an injection every 2 weeks however, any administration of the second agent within these two weeks must be regarded as concomitant. Application form and administration sites are independent; hence, they might be completely different, but as well the two agents might also be provided within the same pharmaceutical composition.

The terms "prophylaxis", "treatment" and "therapy" comprise the administration of at least any substance of the invention, alone or in combination with at least one further substance of the invention and optionally one or more further pharmaceutical drugs to a subject in need thereof, independently of the chronological order of the administration. Such an administration is intended to prevent or inhibit any symptoms or unwanted effects associated with one of the disorders of the invention or with the treatment of such a disorder, and /or to mitigate the unwanted effects or symptoms and/or to initiate a healing process of such a disorder.

Glucocorticoids are, for example, used to treat rheumatoid arthritis, bursitis, osteoarthritis, tendonitis, gouty arthritis, epicondylitis, ulcerative colitis, Crohn's disease, multiple sclerosis, asthma, bronchitis, skin rashes, allergies, severe hypersensitivity reactions, shock, systemic lupus erythematosus, severe psoriasis, alopecia, lichen simplex chronicus, granuloma annulare, granulomatosis with polyangiitis, lichen planus, keloids, acute and chronic leukemias, lymphomas, multiple myeloma, tumors of the brain, intracranial tumors, cerebral edema, idiopathic thrombocytopenic purpura, autoimmune hemolytic anemia, acute mountain sickness, acute idiopathic tinnitus, eczema, muscular dystrophies, Bell's palsy, cluster headaches, chronic obstructive pulmonary disease (COPD), croup, chemotherapy induced fatigue, nausea and vomiting, and to prevent transplant rejection.

Further, early intervention with dexamethasone in a septic shock could reduce the mortality rate, particularly if low doses are used (Lian XJ et al. 2019, Hindawi, BioMed Res Int, Article ID 3175047, published 10 June 2019). Just recently the same effect could be shown in patients with severe Covid-19 symptoms requiring artificial respiration after developing an acute respiratory stress syndrome due to an overreaction of the immune system (Tomazini et al. 2020, JAMA 324(13), 1307-1316; Horby et al. 2020, NEJM 384, 693-704). Hence, glucocorticoid treatments increasing the risk of infections were found to be helpful in critically ill patients at high risk for an infection or the infection itself is even the reason for treatment requirements. Dosing is critical and any of the adverse reactions associated with glucocorticoids could be fatal for these patients. A pharmaceutical combination according to the invention could help to further reduce the administered doses in such cases thereby reducing the adverse reaction risk for the patient.

In the scope of surgeries glucocorticoids are used to reduce inflammation in delicate tissues, e.g., neurosurgery, as glucocorticoids are well-known for the successful treatment of brain edema.

Various skin conditions like, e.g., eczema or reactions to contact poisons such as poison ivy are mainly treated by means of topical applications. In pediatrics, glucocorticoids are primarily used to treat cerebral edema, meningitis, chemotherapy induced nausea and vomiting, acute asthma, croup, acute mountain sickness, idiopathic thrombocytopenic purpura and any further indication that requires a fast and reasonable anti-inflammatory and/or immunosuppressive effect.

Glucocorticoids are also used for prophylaxis, e.g., to prevent exacerbations of COPD or to prevent a posttraumatic acute shock lung.

Also, in the field of veterinary medicine glucocorticoids are widely used to treat allergies and inflammatory diseases of skin, lungs and gastrointestinal tract, but also in the treatment of conditions such as cerebral edema and cardiogenic or septic shock.

The agent or the pharmaceutical combination according to the invention can be used in the in the prophylaxis and/or treatment of unwanted glucocorticoid effects and/or in the treatment of conditions or diseases usually treated with glucocorticoids wherein these conditions or diseases can be selected from a group, without being limiting, that comprises
autoimmune disorders such as multiple sclerosis, rheumatoid arthritis, juvenile chronic arthritis, polymyalgia rheumatica, autoimmune hemolytic anemia, Sjögren syndrome, autoimmune hepatitis, systemic lupus erythematosus, rheumatic fever, alopecia, pemphigus, bullous pemphigoid, dermatomyositis, psoriasis, pyoderma gangrenosum, autoimmune thyroiditis, and immune thrombocytopenia;
allergies, such as asthma, skin rashes, contact dermatitis, e.g. due to poison oak or poison ivy exposure, urticaria, angioedema, anaphylaxis, and severe hypersensitivity reactions;
cancers such as leukemias, including acute and chronic lymphoblastic leukemias as well as acute and chronic myelogenous leukemias, lymphomas, Hodgkin lymphoma, Non-Hodgkin lymphoma, multiple myeloma, tumors of the brain and intracranial tumors;
adverse reactions associated to cancer therapies, such as chemotherapy and radiation therapy, including but not limited to fatigue, nausea and vomiting;
inflammatory diseases of the skin, such as skin rashes, eczema, dermatitis, contact dermatitis, atopic dermatitis, urticaria, systemic lupus erythematosus, alopecia, psoriasis, phimosis, granuloma annulare, pemphigus, bullous pemphigoid, pyoderma gangrenosum, shingles, dermatomyositis, keloids and lichen, such as lichen simplex chronicus, lichen planus, lichen sclerosus and lichen sclerosus et atrophicus;
inflammatory diseases of the respiratory system, such as asthma, bronchitis, laryngitis, croup, severe tuberculosis, chronic obstructive pulmonary disease (COPD), prevention of exacerbations in COPD, cystic fibrosis, lipid pneumonitis, acute respiratory distress syndrome (ARDS) and prophylaxis of infant respiratory distress syndrome (IRDS) as well as of acute shock lung after a trauma;
inflammatory diseases of the gastrointestinal tract, such as inflammatory bowel disease, ulcerative colitis, cystic fibrosis, and Crohn's disease;
inflammatory diseases of the urogenital system, such as acute interstitial nephritis, nephrotic syndrome, cystic fibrosis and phimosis;
inflammatory diseases of the musculoskeletal system, such as bursitis, osteoarthritis, tendonitis, gouty arthritis, epicondylitis, ataxia telangiectasia, plantar fasciitis, mixed connective tissue disease, dermatomyositis, juvenile chronic arthritis, and polymyalgia rheumatica,
muscular dystrophies, such as such as Duchenne muscular dystrophy, Becker muscular dystrophy, limb girdle dystrophies, dysferlinopathies, Myoshi myopathy 1-3, hereditary inclusion body myopathy (HIBM), distal myopathy with rimmed vacuoles (DMRV), Nonaka distal myopathy, quadriceps-sparing myopathy, spinal and bulbar muscular atrophy (SMA), SMARD1, Werdnig-Hoffmann disease, Kugelberg-Welander disease, Charcot-Marie-Tooth disease, Curschmann-Steinert's disease, proximal myotonic myopathy, PROMM (type 2), Walker-Warburg syndrome, lamin A/C-related congenital muscular dystrophy, Fukuyama congenital muscular dystrophy, congenital muscular dystrophy with partial merosin deficiency, rigid spine muscular dystrophy, congenital muscular dystrophy with primary laminin 2 deficiency, LARGE-related congenital muscular dystrophy, muscle-eye-brain disease, Ullrich congenital muscular dystrophy, Emery-Dreyfuss muscular dystrophy (EMD), facioscapulohumeral muscular dystrophy, oculopharyngeal muscular dystrophy and myofibrillar myopathies types 1 - 6;
inflammatory diseases of nervous system and sensory organs, such as ocular inflammation, sympathetic ophthalmia, uveitis, meningitis, cluster headaches, ataxia telangiectasia, Bell's palsy, shingles, chronic inflammatory demyelinating polyneuropathy (CIPD), myasthenia gravis, Menière's disease, acute idiopathic tinnitus,
inflammatory diseases of the cardiovascular system, such as heart failure, pericarditis, postural hypotension, rheumatic fever with carditis, vasculitis, polyarteritis nodosa, panarteritis nodosa, giant-cell arteritis, temporal arteritis, and granulomatosis with polyangiitis;
further systemic inflammatory diseases, such as sepsis, including but not limited to septic shock, bacteremia, viremia, fungemia, sepsis associated to parasites, Herxheimer reaction, and SIRS (systemic inflammatory response syndrome) as well as further inflammatory response syndromes, such as the acute respiratory distress syndrome (ARDS), the Kawasaki syndrome and the pediatric inflammatory multisystem syndrome temporally associated with SARS-CoV-2" (PIMS-TS);
severe infectious diseases, such as tuberculosis, typhus and brucellosis;
and a great number of further conditions, such as cerebral edema, shock; posttraumatic shock syndrome, posttraumatic stress disorder, sarcoidosis, hypercalcemia, adrenal insufficiency, including primary, secondary and tertiary adrenal insufficiency, adrenogenital syndrome, thyroiditis, and acute mountain sickness;
and to prevent transplant rejection.

Surprisingly, it could be shown that the concomitant administration of a pharmaceutical combination according to the invention not only shows a general supraadditive effect, but also shows this effect over the whole great range of fixed ratios tested (see Example 1).

The term "ratio" or "fixed ratio" hereby refers to any kind of ratio between two components independent of the units used. Such a ratio is valid for weight, weight %, concentration specifications and any other feasible unit in the field of pharmaceuticals. Hence, for example a ratio of the glucocorticoid used to the 5-amino-2,3-dihydro-1,4-phthalazinedione used of 1:10 could for example be implemented as 1mg:10mg, 1mg/kg:10mg/kg, 1mM/10mM or 1%:10% or in any other unit as long as the ratio itself is 1:10.

The present patent application also refers to a glucocorticoid-sparing agent or a respective pharmaceutical combination comprising at least one 5-amino-2,3-dihydro-1,4-phthalazinedione and at least one glucocorticoid for use in the prophylaxis and/or treatment of conditions or diseases usually treated with glucocorticoids, wherein the efficacy of the prophylaxis and / or treatment is significantly improved compared to the respective treatment with glucocorticoids alone.

Further, the glucocorticoid-sparing agent or the respective pharmaceutical combination according to the invention can thus be used in the prophylaxis and/or treatment of conditions or diseases usually treated with glucocorticoids, wherein the components can be used in any ratio.

However, to receive the best effects the medical indication to be treated, the potency of the glucocorticoid to be used, application form and feasibility of the ratio from a practical point of view should be considered, the latter in particular to maintain patient compliance.

The glucocorticoid-sparing agent or the respective pharmaceutical combination according to the invention can thus be used in the prophylaxis and/or treatment of conditions or diseases usually treated with glucocorticoids, wherein the components glucocorticoid and 5-amino-2,3-dihydro-1,4-phthalazinedione can be preferably used in any ratio from 10:1 to 1:5000, more preferably from 4:1 to 1:1600, most preferably from 1:5 to 1:400.

In the following some possible combinations and respective ratios are provided, however, the pharmaceutical combinations according to the invention are not limited to these examples.

Depending on application form, indication to be treated and patients personal risk situation dexamethasone for oral application for example is administered in doses of up to 100 mg per day in severe and acute cases whereas maintenance doses in chronic conditions usually vary between 4 and 8 mg per day. 5-amino-2,3-dihydro-1,4-phthalazinedione has been shown to be very safe, however, due to compliance reasons dosages should not exceed 10 grams per day in case of, e.g., tablets or capsules.

The glucocorticoid-sparing agent or the respective pharmaceutical combination according to the invention can thus be used in the prophylaxis and/or treatment of conditions or diseases usually treated with glucocorticoids, wherein the component dexamethasone is administered orally, characterized by a ratio of dexamethasone to 5-amino-2,3-dihydro-1,4-phthalazinedione sodium salt from 4:1 to 1:100, preferably 2:1 to 1:50, more preferably 1:1 to 1:20, most preferably 1:2 to 1:10 in severe acute cases, and characterized by a ratio of dexamethasone to 5-amino-2,3-dihydro-1,4-phthalazinedione sodium salt from 1:5 to 1:800, preferably 1:10 to 1:400, more preferably 1:20 to 1:200, most preferably 1:50 to 1:100 for maintenance doses in chronic cases.

However, in case of liquid applications higher doses and/or amounts could be administered without negative impact on patient's compliance.

Thus, the glucocorticoid-sparing agent or the respective pharmaceutical combination according to the invention can be used in the prophylaxis and/or treatment of conditions or diseases usually treated with glucocorticoids, wherein the components dexamethasone and 5-amino-2,3-dihydro-1,4-phthalazinedione sodium salt are administered orally, characterized by a ratio of low-dosed dexamethasone to 5-amino-2,3-dihydro-1,4-phthalazinedione sodium salt from 1:5 to 1:1600, preferably 1:10 to 1:800, more preferably 1:20 to 1:400, most preferably 1:50 to 1:200 in patients receiving their medicaments in any liquid form.

Dexamethasone for injection is dosed quite similar compared to the oral administration. However, in very severe diseases acute initial doses up to 200 mg are injected intravenously and also maintenance doses are higher in these cases. Doses for local infiltration and injection therapies usually vary between 2 mg and 8 mg per injection.

The glucocorticoid-sparing agent or the respective pharmaceutical combination according to the invention can thus be used in the prophylaxis and/or treatment of conditions or diseases usually treated with glucocorticoids, wherein the component dexamethasone is administered intravenously, characterized by a ratio of dexamethasone to 5-amino-2,3-dihydro-1,4-phthalazinedione sodium salt from 10:1 to 1:100, preferably 4:1 to 1:50, more preferably 2:1 to 1:20, most preferably 1:1 to 1:10 in severe acute cases, and characterized by a ratio of dexamethasone to 5-amino-2,3-dihydro-1,4-phthalazinedione sodium salt from 1:1 to 1:400, preferably 1:2 to 1:200, more preferably 1:5 to 1:100, most preferably 1:10 to 1:50 for maintenance doses in chronic cases.

The glucocorticoid-sparing agent or the respective pharmaceutical combination according to the invention can thus be used in the prophylaxis and/or treatment of conditions or diseases usually treated with glucocorticoids, wherein the component dexamethasone is locally administered via infiltration or injection, characterized by a ratio of dexamethasone to 5-amino-2,3-dihydro-1,4-phthalazinedione sodium salt from 1:5 to 1:1600, preferably 1:10 to 1:800, more preferably 1:20 to 1:400, most preferably 1:50 to 1:200.

Prednisone when given orally starts from low maintenance doses of 1 mg per day up to 250 mg per day in severe acute cases.

The glucocorticoid-sparing agent or the respective pharmaceutical combination according to the invention can thus be used in the prophylaxis and/or treatment of conditions or diseases usually treated with glucocorticoids, wherein the component prednisone is administered orally, characterized by a ratio of dexamethasone to 5-amino-2,3-dihydro-1,4-phthalazinedione sodium salt from 10:1 to 1:100, preferably 4:1 to 1:50, more preferably 2:1 to 1:20, most preferably 1:1 to 1:10 in severe acute cases, and characterized by a ratio of prednisone to 5-amino-2,3-dihydro-1,4-phthalazinedione sodium salt from 1:2 to 1:1600, preferably 1:5 to 1:800, more preferably 1:10 to 1:400, most preferably 1:20 to 1:200 for maintenance doses in chronic cases.

Prednisolone as injection in severe acute cases is administered in doses up to 1000 mg. Maintenance doses of 50 mg or more per day are not uncommon.

The glucocorticoid-sparing agent or the respective pharmaceutical combination according to the invention can thus be used in the prophylaxis and/or treatment of conditions or diseases usually treated with glucocorticoids, wherein the component prednisolone is administered intravenously, characterized by a ratio of prednisolone to 5-amino-2,3-dihydro-1,4-phthalazinedione sodium salt from 10:1 to 1:20, preferably 4:1 to 1:10, more preferably 2:1 to 1:5, most preferably 1:1 to 1:2 in severe acute cases, and characterized by a ratio of dexamethasone to 5-amino-2,3-dihydro-1,4-phthalazinedione sodium salt from 4:1 to 1:400, preferably 2:1 to 1:200, more preferably 1:1 to 1:100, most preferably 1:2 to 1:50 for maintenance doses.

For further glucocorticoid substances as well as for further application forms such as such as creams for topical treatments or preparations for inhalation comprising glucocorticoids suitable ratios can be determined accordingly.

Due to the supraadditivity shown for the pharmaceutical combinations of the invention the glucocorticoid dose can be reduced depending on individual patient, indication and ratio of components used. In some cases, dose and ratio adaption over time might become necessary to receive best results. As shown in Example 1 (cf. table 2) improvements due to the combination compared to the glucocorticoid alone reach 25% in nearly all combinations investigated, improvements of 50% or even 100% are very common, improvements of up to more than 500% had been observed in single combinations.

Thus, the glucocorticoid dosage in the pharmaceutical combination of the invention can be reduced to 80%, preferably 50%, most preferably 20% compared to the dosage when the glucocorticoid is administered alone.

The present application refers thus to 5-amino-2,3-dihydro-1,4-phthalazinedione or one of its pharmaceutically acceptable salts for use as glucocorticoid-sparing agent in the prophylaxis and/or treatment of conditions usually treated with glucocorticoids, characterized in that the dose of the glucocorticoid can be significantly reduced. Such a significant reduction means a reduction of the glucocorticoid dose to 80%, preferably 50%, most preferably 20%, of the original dose.

The present patent application also refers to a pharmaceutical combination comprising at least one 5-amino-2,3-dihydro-1,4-phthalazinedione and at least one glucocorticoid for use in the prophylaxis and/or treatment of conditions or diseases usually treated with glucocorticoids, wherein the dose of the glucocorticoid can be significantly reduced compared to the respective treatment with glucocorticoids alone. Such a significant reduction means a reduction of the glucocorticoid dose to 80%, preferably 50%, most preferably 20%, of the original dose.

By reducing the glucocorticoid dose respective unwanted effects will occur more seldomly and less intensely. Further, Example 2 gives a clear hint, that unwanted side effects of glucocorticoids will be diminished when the pharmaceutical combination of the invention is administered, even if the glucocorticoid dose was not reduced.

The present patent application also refers to a pharmaceutical combination comprising at least one 5-amino-2,3-dihydro-1,4-phthalazinedione and at least one glucocorticoid for use in the prophylaxis and/or treatment of conditions or diseases usually treated with glucocorticoids, wherein the number and severity of glucocorticoid related side effects is significantly reduced compared to the respective treatment with glucocorticoids alone.

Depending on the specific glucocorticoid used, there may arise a problem with high administered dosages, since above a critical serum concentration not only the glucocorticoid receptor is activated but to a lesser degree also the mineralocorticoid receptor. In most cases this is an unwanted side effect. By an application according to the invention of a combination of a glucocorticoid and 5-amino-2,3-dihydro-1,4-phthalazinedione or one of its pharmaceutically acceptable salts the effective dosage of this glucocorticoid can be reduced so that no activation of the mineralocorticoid receptor occurs, or at least to a much lesser degree. For attaining this effect, the use of 5-amino-2,3-dihydro-1,4-phthalazinedione sodium salt is particularly preferred.

The present patent application refers to a method of treatment by providing a pharmaceutical combination comprising at least one 5-amino-2,3-dihydro-1,4-phthalazinedione and at least one glucocorticoid for use in the prophylaxis and/or treatment of conditions or diseases usually treated with glucocorticoids, to a patient in need thereof. The method comprises the provision of the pharmaceutical combination of the invention by administering said combination in an effective amount and ratio to a patient in need thereof. Examples of such effective amounts and ratios have been described above.

The present patent application also refers to a method of treatment by providing a pharmaceutical combination comprising at least one 5-amino-2,3-dihydro-1,4-phthalazinedione and at least one glucocorticoid for use in the prophylaxis and/or treatment of unwanted glucocorticoid effects, to a patient in need thereof. The method comprises the provision of the pharmaceutical combination of the invention; by administering said combination in an effective amount and ratio to a patient in need thereof. Examples of such effective amounts and ratios have been described above.

The present application refers likewise to a pharmaceutical composition for use as add-on in the prophylaxis and/or treatment of conditions or diseases usually treated with glucocorticoids, wherein said composition contains at least one 5-amino-2,3-dihydro-1,4-phthalazinedione and at least one excipient.

The present application also refers to a pharmaceutical composition for use in the prophylaxis and/or treatment of conditions or diseases usually treated with glucocorticoids, wherein said composition contains at least one of 5-amino-2,3-dihydro-1,4-phthalazinedione or any of aforementioned molecular variants of this compound, at least one glucocorticoid and at least one excipient.

The at least one excipient can be selected from a group comprising carriers, penetration enhancers; binding agents; solvents; solubilizing agents; buffers; preservatives; antioxidants; coatings; colorants; flavoring substances; aromatic substances; sweeteners; thickening agents; disintegrants; glidants; lubricants; emulsifiers; stabilizers; diluents; anti-caking agents (antiadherents); pH regulators, acidifiers, isotonizing agents, foaming agents, anti-foaming agents, fatliquors, consistency enhancers, hydrotropes, sorbents and opacifiers.

Eligible carriers are all carriers known in the art and combinations thereof. In solid dosage forms they can be for example plant and animal fats, waxes, paraffins, starch, tragacanth, cellulose derivatives, polyethylene glycols, silicones, bentonites, silica, talcum, zinc oxide. For liquid dosage forms and emulsions suitable carriers are for example solvents, solubilizing agents, emulsifiers such as water, ethanol, isopropanol, ethyl carbonate, ethyl acetate, benzyl alcohol, benzyl benzoate, propylene glycol, 1,3-butyl glycol, cotton seed oil, peanut oil, olive oil, castor oil, sesame oil, glycerol fatty acid esters, polyethylene glycols, fatty acid esters of sorbitan. Suspensions may use carriers known in the art such as diluents (e.g., water, ethanol or propylene glycol), ethoxylized isostearyl alcohols, polyoxyethylene and polyoxyethylene sorbitan esters, microcrystalline cellulose, bentonites, agar agar, tragacanth.

Permeation enhancers are often used in topical dosage forms. Suitable permeation enhancers comprise all pharmaceutically acceptable permeation enhancers known in the art, such as, without being limiting, azones such as laurocapran, 1-dodecylazacycloheptan-2-one; sulphoxides such as dimethylsulphoxide, DMAC, DMF; pyrrolidones such as 2-pyrrolidone, N-methyl-2-pyrrolidone; alcohols such as ethanol, 1,2-propandiol or decanol; glycols such as propylene glycol, diethylene glycol, tetraethylene glycol; fatty acids such as oleic acid, lauric acid, sodium lauryl sulfate, myristic acid, isopropyl myristic acid, capric acid; nonic surfactants such as polyoxyethylene-2-oleyl ether, polyoxyethylene-2-stearyl ether; terpenes; terpenoids; oxazolidinones; urea; ceramide analogs, azone analogs, menthol derivatives, etherified derivatives, esterified derivatives, transcarbams, carbamate salts, TXA derivatives, DDAIP (dodecyl 2-(dimethylamino) propanoate), DDAK, natural essential oils (all of them listed in Chen et al. (2014) Asian J. Pharm. Sc. 9, 51-64); citric acid esters such as triethyl citrate; hydrophobin polypeptides; alpha-bisabolol; dimethyl isosorbide (Arlasove^{®} DMI); ethoxydiglycol. Preferred is 1,2-propandiol.

The term binding agents refers to substances that bind powders or glue them together, rendering them cohesive through granule formation. They serve as a "glue" of the formulation. Binding agents increase the cohesive strength of the provided diluent or filler.

Suitable binding agents are starch from wheat, corn, rice or potato, gelatin, naturally occurring sugars such as glucose, sucrose or beta-lactose, sweeteners from corn, natural and synthetic gums such as acacia, tragacanth or ammonium calcium alginate, sodium alginate, carboxymethyl cellulose, sodium carboxymethyl cellulose, hydroxypropyl carboxymethyl cellulose, polyethylene glycol, polyvinyl pyrrolidone, magnesium aluminum silicate, waxes and others. The percentage of the binding agent in the composition can range from 1 - 30 % by weight, preferred 2 - 20 % by weight, more preferred 3 - 10 % by weight and most preferred 3 - 6 % by weight.

Suitable solvents may be selected from the group comprising water, carbonated water, water for injection, water with isotonizing agents, saline, isotonic saline, alcohols, particularly ethyl and n-butyl alcohol, glycols, oleic and linoleic acid triglycerides, caprylic and capric acid mono-, di- and triglycerides, polyoxyethylene caprylic and capric acid glycerides, propylene glycol fatty acid esters, low alkyl fatty acid esters, soy bean oil, propylene glycol laurate, polyoxyethylene 35 (peg-35) castor oil, polyoxyethylene glyceryl trioleate, ethyl butyrate, ethyl caprylate, ethyl oleate and mixtures thereof.

Suitable as surface-active solubilizing agents (solubilizers) are for example diethylene glycol monoethyl ester, polyethyl propylene glycol co-polymers, cyclodextrins such as □- and □-cyclodextrin, glyceryl monostearates such as Solutol HS 15 (macrogol-15-hydroxystearate from BASF, PEG 660-15 hydroxystearates), sorbitan esters, polyoxyethylene glycol, polyoxyethylene sorbitanic acid esters, polyoxyethylene sorbitan monooleate, polyoxyethylene oxystearic acid triglyceride, polyvinyl alcohol, sodium dodecyl sulfate, (anionic) glyceryl monooleates etc.

Moreover, buffers or buffer solutions are preferred for liquid formulations, in particular for pharmaceutical liquid formulations. The terms buffer, buffer system and buffer solution, in particular of an aqueous solution, refer to the capacity of the system to resist a pH change by the addition of an acid or a base, or by dilution with a solvent. Preferred buffer systems may be selected from the group comprising formate, lactate, benzoic acid, oxalate, fumarate, aniline, acetate buffer, citrate buffer, glutamate buffer, phosphate buffer, succinate, pyridine, phthalate, histidine, MES (2-(N-morpholino) ethanesulfonic acid, maleic acid, cacodylate (dimethyl arsenate), carbonic acid, ADA (N-(2-acetamido)imino diacetic acid, PIPES (4-piperazine-bis-ethanesulfonic acid), BIS-TRIS propane (1,3-bis[tris(hydroxymethyl)mehylaminol] propane), ethylene diamine, ACES (2-[(amino-2-oxoethyl)amino]ethanesulfonic acid), imidazole, MOPS (3-(N-morphino)-propanesulfonic acid, diethyl malonic acid, TES (2-[tris(hydroxymethyl)methyl]aminoethanesulfonic acid, HEPES (N-2-hydroxyethylpiperazine-N'-2-ethanesulfonic acid), as well as other buffers with a pKₐ between 3.8 and 7.7.

Preferred are carbonic acid buffers such as acetate buffer and dicarboxylic acid buffers such as fumarate, tartrate and phthalate as well as tricarboxylic acid buffers such as citrate.

A further group of preferred buffers are inorganic buffers such as sulfate hydroxide, borate hydroxide, carbonate hydroxide, oxalate hydroxide, calcium hydroxide and phosphate buffers. Another group of preferred buffers are nitrogen-containing puffers such as imidazole, diethylene diamine and piperazine. Furthermore, preferred are sulfonic acid buffers such as TES, HEPES, ACES, PIPES, [(2-hydroxy-1,1-bis-(hydroxymethyl)ethyl)amino]-1-propanesulfonic acid (TAPS), 4-(2-hydroxyethyl)piperazine-1-propanesulfonic acid (EEPS), 4-morpholinopropanesulfonic acid (MOPS) and N,N-bis-(2-hydroxyethyl)-2-aminoethanesulfonic acid (BES). Another group of preferred buffers are glycine, glycyl-glycine, glycyl-glycyl-glycine, N,N-bis-(2-hydroxyethyl)-glycine and *N*-[2-hydroxy-1,1-bis(hydroxymethyl)ethyl]glycine (tricine). Preferred are also amino acid buffers such as glycine, alanine, valine, leucine, isoleucine, serine, threonine, phenylalanine, tyrosine, tryptophan, lysine, arginine, histidine, aspartate, glutamate, asparagine, glutamine, cysteine, methionine, proline, 4-hydroxy proline, *N,N,N-*trimethyllysine, 3-methyl histidine, 5-hydroxy-lysine, o-phosphoserine, gamma-carboxyglutamate, [epsilon]-N-acetyl lysine, [omega]-N-methyl arginine, citrulline, ornithine and their derivatives.

Preservatives for liquid dosage forms can be used on demand. They may be selected from the group comprising sorbic acid, potassium sorbate, sodium sorbate, calcium sorbate, methyl paraben, ethyl paraben, methyl ethyl paraben, propyl paraben, benzoic acid, sodium benzoate, potassium benzoate, calcium benzoate, heptyl p-hydroxybenzoate, sodium methyl para-hydroxybenzoate, sodium ethyl para-hydroxybenzoate, sodium propyl para-hydroxybenzoate, benzyl alcohol, benzalkonium chloride, phenylethyl alcohols, cresols, cetylpyridinium chloride, chlorobutanol, thiomersal (sodium 2-(ethylmercurithio) benzoic acid), sulfur dioxide, sodium sulfite, sodium bisulfite, sodium metabisulfite, potassium metabisulfite, potassium sulfite, calcium sulfite, calcium hydrogen sulfite, potassium hydrogen sulfite, biphenyl, orthophenyl phenol, sodium orthophenyl phenol, thiabendazole, nisin, natamycin, formic acid, sodium formate, calcium formate, hexamine, formaldehyde, dimethyl dicarbonate, potassium nitrite, sodium nitrite, sodium nitrate, potassium nitrate, acetic acid, potassium acetate, sodium acetate, sodium diacetate, calcium acetate, ammonium acetate, dehydroacetic acid, sodium dehydroacetate, lactic acid, propionic acid, sodium propionate, calcium propionate, potassium propionate, boric acid, sodium tetraborate, carbon dioxide, malic acid, fumaric acid, lysozyme, copper-(II)-sulfate, chlorine, chlorine dioxide and other suitable substances or compositions known to the person skilled in the art.

The addition of antioxidants is particularly preferable in topical dosage forms. Suitable examples for antioxidants include sodium metabisulfite, alpha-tocopherol, ascorbic acid, maleic acid, sodium ascorbate, ascorbyl palmitate, butylated hydroxyanisol, butylated hydroxytoluol, fumaric acid or propyl gallate. Preferred is the use of sodium metabisulfite.

Tablets or pills are usually coated, i.e. the coating constitutes the outer layer. This can be a film coating, a sugar coating with saccharides and a compression coating. Pharmaceutically acceptable varnishes or waxes, HPMC, MC or HPC can be used. Such a coating may help to disguise the taste, to ease the swallowing or the identification. Often plasticizers and pigments are included in the coating.

Capsules normally have a gelatinous envelope that encloses the active substance. The specific composition and thickness of this gelatinous layer determines how fast absorption takes place after ingestion of the capsule. Of special interest are sustained release formulations, as known in the art. Colorants are excipients that bestow a colorization to the composition of the drink, respectively the dosage form. These excipients can be food colorants. They can be adsorbed on a suitable adsorption means such as clay or aluminum oxide. The amount of the colorant may vary between 0.01 and 10 % per weight of the composition, preferred between 0.05 and 6 % per weight, more preferred between 0.1 and 4 % per weight, most preferred between 0.1 and 1 % per weight. Suitable food colorants are curcumin, riboflavin, riboflavin-5'-phosphate, tartrazine, alkanin, quinolione yellow WS, Fast Yellow AB, riboflavin-5'-sodium phosphate, yellow 2G, Sunset yellow FCF, orange GGN, cochineal, carminic acid, citrus red 2, carmoisine, amaranth, Ponceau 4R, Ponceau SX, Ponceau 6R, erythrosine, red 2G, Allura red AC, Indathrene blue RS, Patent blue V, indigo carmine, Brilliant blue FCF, chlorophylls and chlorophyllins, copper complexes of chlorophylls and chlorophyllins, Green S, Fast Green FCF, Plain caramel, Caustic sulfite caramel, ammonia caramel, sulphite ammonia caramel, Black PN, Carbon black, vegetable carbon, Brown FK, Brown HT, alpha-carotene, beta-carotene, gamma-carotene, annatto, bixin, norbixin, paprika oleoresin, capsanthin, capsorubin, lycopene, beta-apo-8'-carotenal, ethyl ester of beta-apo-8'-carotenic acid, flavoxanthin, lutein, cryptoxanthin, rubixanthin, violaxanthin, rhodoxanthin, canthaxanthin, zeaxanthin, citranaxanthin, astaxanthin, betanin, anthocyanins, saffron, calcium carbonate, titanium dioxide, iron oxides, iron hydroxides, aluminum, silver, gold, pigment rubine, tannin, orcein, ferrous gluconate, ferrous lactate.

Suitable aromatic and flavoring substances comprise above all essential oil that can be used for this purpose. In general, this term refers to volatile extracts from plants or parts of plants with the respective characteristic smell. They can be extracted from plants or parts of plants by steam distillation. Examples are: Essential oils, respectively aromatic substances from sage, cloves, chamomile, anise, star anise, thyme, tea tree, peppermint, mint oil, menthol, cineol, eucalyptus oil, mango, figs, lavender oil, chamomile blossoms, pine needles, cypress, oranges, rosewood, plum, currant, cherry, birch leaves, cinnamon, limes, grapefruit, tangerine, juniper, valerian, lemon balm, lemon grass, palmarosa, cranberry, pomegranate, rosemary, ginger, pineapple, guava, echinacea, ivy leave extract, blueberry, kaki, melons etc. or mixtures thereof, as well as mixtures of menthol, peppermint and star anise oil or menthol and cherry flavor. These aromatic or flavoring substances can be included in the range of 0.0001 to 10 % per weight (particularly in a composition), preferred 0.001 to 6% per weight, more preferred 0.001 to 4% per weight, most preferred 0.01 to 1% per weight, with regard to the total composition. Application- or single case-related it may be advantageous to use differing quantities.

Suitable sweeteners can be selected from the group comprising mannitol, glycerol, acesulfame potassium, aspartame, cyclamate, isomalt, isomaltitol, saccharin and its sodium, potassium and calcium salts, sucralose, alitame, thaumatin, glycyrrhizin, neohesperidine dihydrochalcone, steviol glycosides, neotame, aspartame-acesulfame salt, maltitol, maltitol syrup, lactitol, xylitol, erythritol.

Suitable thickening agents can be selected from the group comprising polyvinyl pyrrolidone, methyl cellulose, hydroxypropyl methyl cellulose, hydroxypropyl cellulose, dextrins, polydextrose, modified starch, alkaline modified starch, bleached starch, oxidized starch, enzymetreated starch, monostarch phosphate, distarch phosphate esterified with sodium trimetaphosphate or phosphorus oxychloride, phosphate distarch phosphate, acetylated distarch phosphate, starch acetate esterified with acetic anhydride, starch acetate esterified with vinyl acetate, acetylated distarch adipate, acetylated distarch glycerol, distarch glycerin, hydroxy propyl starch, hydroxy propyl distarch glycerin, hydroxy propyl distarch phosphate, hydroxy propyl distarch glycerol, starch sodium octenyl succinate, acetylated oxidized starch, hydroxyethyl cellulose.

Suitable disintegrants can be selected from the group comprising starch, cold water-soluble starches such as carboxymethyl starch, cellulose derivatives such as methyl cellulose and sodium carboxymethyl cellulose, microcrystalline cellulose and cross-linked microcrystalline celluloses such as croscarmellose sodium, natural and synthetic gums such as guar, agar, karaya (Indian tragacanth), locust bean gum, tragacanth, clays such as bentonite, xanthan gum, alginates such as alginic acid and sodium alginate, foaming compositions a.o. Moisture expansion is supported by for example starch, cellulose derivatives, alginates, polysaccharides, dextrans, cross-linked polyvinyl pyrrolidone. The amount of the disintegrant in the composition may vary between 1 and 40 % per weight, preferred between 3 and 20% per weight, most preferred between 5 and 10 % per weight.

Glidants are materials that prevent a baking of the respective supplements and improve the flow characteristics of granulations so that the flow is smooth and constant. Suitable glidants comprise silicon dioxide, magnesium stearate, sodium stearate, starch and talcum. The amount of the glidant in the composition may vary between 0.01 and 10 % per weight, preferred between 0.1 and 7 % per weight, more preferred between 0.2 and 5 % per weight, most preferred between 0.5 and 2 % per weight.

The term lubricants refers to substances that are added to the dosage form in order to facilitate tablets, granulates etc. to be released from the press mold or the outlet nozzle. They diminish friction or abrasion. Lubricants are usually added shortly before pressing, as they should be present on the surface of the granules and between them and the parts of the press mold. The amount of the lubricant in the composition may vary between 0.05 and 15 % per weight, preferred between 0.2 and 5 % per weight, more preferred between 0.3 and 3 % per weight, most preferred between 0.3 and 1.5 % per weight. Suitable lubricants are a.o. sodium oleate, metal stearates such as sodium stearate, calcium stearate, potassium stearate and magnesium stearate, stearic acid, sodium benzoate, sodium acetate, sodium chloride, boric acid, waxes having a high melting point, polyethylene glycol.

Emulsifiers can be selected for example from the following anionic and non-ionic emulsifiers: Anionic emulsifier waxes, cetyl alcohol, cetylstearyl alcohol, stearic acid, oleic acid, polyoxyethylene polyoxypropylene block polymers, addition products of 2 to 60 mol ethylene oxide to castor oil and/or hardened castor oil, wool wax oil (lanolin), sorbitan esters, polyoxyethylene alkyl esters, polyoxyethylene sorbitan fatty acid esters, polyoxyethene sorbitan monolaurate, polyoxyethene sorbitan monooleate, polyoxyethene sorbitan monopalmitate, polyoxyethene sorbitan monostearate, polyoxyethene sorbitan tristearate, polyoxyethene stearate, polyvinyl alcohol, metatartaric acid, calcium tartrate, alginic acid, sodium alginate, potassium alginate, ammonium alginate, calcium alginate, propane-1,2-diol alginate, carrageenan, processed eucheuma seaweed, locust bean gum, tragacanth, acacia gum, karaya gum, gellan gum, gum ghatti, glucomannane, pectin, amidated pectin, ammonium phosphatides, brominated vegetable oil, sucrose acetate isobutyrate, glycerol esters of wood rosins, disodium phosphate, trisodium diphosphate, tetrasodium diphosphate, dicalcium diphosphate, calcium dihydrogen diphosphate, sodium triphosphate, pentapotassium triphosphate, sodium polyphosphates, sodium calcium polyphosphate, calcium polyphosphates, ammonium polyphosphate, beta-cyclodextrin, powdered cellulose, methyl cellulose, ethyl cellulose, hydroxypropyl cellulose, hydroxypropyl methylcellulose, ethyl methyl cellulose, carboxymethyl cellulose, sodium carboxymethyl cellulose, ethyl hydroxyethyl cellulose, croscarmellose, enzymically hydrolyzed carboxymethyl cellulose, mono- and diglycerides of fatty acids, glyceryl monostearate, glyceryl distearate, acetic acid esters of mono- and diglycerides of fatty acids, lactic acid esters of mono- and diglycerides of fatty acids, citric acid esters of mono- and diglycerides of fatty acids, tartaric acid esters of mono- and diglycerides of fatty acids, mono- and diacetyl tartaric acid esters of mono- and diglycerides of fatty acids, mixed acetic and tartaric acid esters of mono- and diglycerides of fatty acids, succinylated monoglycerides, sucrose esters of fatty acids, sucroglycerides, polyglycerol esters of fatty acids, polyglycerol polyricinoleate, propane-1,2-diol esters of fatty acids, propylene glycol esters of fatty acids, lactylated fatty acid esters of glycerol and propane-1, thermally oxidized soy bean oil interacted with mono- and diglycerides of fatty acids, dioctyl sodium sulphosuccinate, sodium stearoyl-2-lactylate, calcium stearoyl-2-lactylate, stearyl tartrate, stearyl citrate, sodium stearoyl fumarate, calcium stearoyl fumarate, stearyl tartrate, stearyl citrate, sodium stearoyl fumarate, calcium stearoyl fumarate, sodium laurylsulfate, ethoxylated mono- and diglycerides, methyl glucoside-coconut oil ester, sorbitan monostearate, sorbitan tristrearate, sorbitan monolaurate, sorbitan monooleate, sorbitan monopalmitate, sorbitan trioleate, calcium sodium polyphosphate, calcium polyphosphate, ammonium polyphosphate, cholic acid, choline salts, distarch glycerol, starch sodium octenyl succinate, acetylated oxidized starch. Preferred are glycerin monooleate, stearic acid, phospholipids such as lecithin.

Stabilizers are substances that can be added to prevent unwanted changes active substances or in other supplements. Though stabilizers are not real emulsifiers they may also contribute to the stability of emulsions. Suitable examples for stabilizers are oxystearin, xanthan gum, agar, oat gum, guar gum, tara gum, polyoxyethene stearate, aspartame-acesulfame salt, amylase, proteases, papain, bromelain, ficin, invertase, polydextrose, polyvinyl pyrrolidone, polyvinyl polypyrrolidone, triethyl citrate, maltitol, maltitol syrup.

Diluents or fillers are inactive substances added to drugs in order to handle minimal amounts of active agents. Examples for suitable diluents are water, mannitol, pre-gelatinized starch, starch, microcrystalline cellulose, powdered cellulose, silicified microcrystalline cellulose, dibasic calcium phosphate dihydrate, calcium phosphate, calcium carbonate, hydroxypropyl cellulose, hydroxyethyl cellulose, hydroxypropyl methylcellulose, polyethylene glycol, xanthum gum, gum arabic or any combination thereof.

Anti-caking agents (antiadherents) can be added to a supplement or a composition of supplements in order to prevent the formation of lumps and for easing packaging, transport, release from the at least one chamber of the dispensing cap and consumption. Suitable examples include tricalcium phosphate, powdered cellulose, magnesium stearate, sodium bicarbonate, sodium ferrocyanide, potassium ferrocyanide, calcium ferrocyanide, bone phosphate, sodium silicate, silicon dioxide, calcium silicate, magnesium trisilicate, talcum powder, sodium aluminosilicate, potassium aluminum silicate, calcium aluminosilicate, bentonite, aluminum silicate, stearic acid, polydimethyl siloxane.

Suitable pH-regulators for liquid dosage forms are e.g. sodium hydroxide, hydrochloric acid, buffer substances such as sodium dihydrogen phosphate or disodium hydrogenphosphate.

Suitable acidity regulators can be selected from the group comprising acetic acid, potassium acetate, sodium acetate, sodium diacetate, calcium acetate, carbon dioxide, malic acid, fumaric acid, sodium lactate, potassium lactate, calcium lactate, ammonium lactate, magnesium lactate, citric acid, mono-, di-, trisodium citrate, mono-, di-, tripotassium citrate, mono-, di-, tricalcium citrate, tartaric acid, mono-, disodium tartrate, mono-, dipotassium tartrate, sodium potassium tartrate, ortho-phosphoric acid, lecithin citrate, magnesium citrate, ammonium malate, sodium malate, sodium hydrogen malate, calcium malate, calcium hydrogen malate, adipic acid, sodium adipate, potassium adipate, ammonium adipate, succinic acid, sodium fumarate, potassium fumarate, calcium fumarate, ammonium fumarate, 1,4-heptonolactone, triammonium citrate, ammonium ferric citrate, calcium glycerophosphate, isopropyl citrate, potassium carbonate, potassium bicarbonate, ammonium carbonate, ammonium bicarbonate, magnesium carbonate, magnesium bicarbonate, ferrous carbonate, ammonium sulfate, aluminum potassium sulfate, aluminum ammonium sulfate, sodium hydroxide, potassium hydroxide, ammonium hydroxide, magnesium hydroxide, gluconic acid.

Acidifiers use to be inorganic chemicals that either produce or become acid. Suitable examples are: Ammonium chloride, calcium chloride.

Suitable isotonizing agents are for example pharmaceutically acceptable salts, in particular sodium chloride and potassium chloride, sugars such as glucose or lactose, sugar alcohols such as mannitol and sorbitol, citrate, phosphate, borate and mixtures thereof.

In some galenic formulations it may be desirable that a liquid oral dosage form generates some foam on being dissolved. Such an effect can be supported through the addition of a foaming agent that reduces the surface tension of the liquid, thus facilitating the formation of bubbles, or it increases its colloidal stability by inhibiting coalescence of bubbles. Alternatively, it may stabilize foam. Suitable examples include mineral oil, quillaia extract, triethyl citrate, sodium lauryl ether sulfate, sodium lauryl sulfate, ammonium lauryl sulfate.

Alternatively, some liquid oral dosage forms may appear slightly foamy upon preparation. Though this does not interfere with the desired application it may affect patient compliance in case of a medication or the commercial success in case of dietary supplements. Therefore, it may be desirable to add a pharmaceutically acceptable anti-foaming agent (defoamer). Examples are polydimethylsiloxane or silicone oil in dietary supplements or simethicone in pharmaceuticals. Suitable fatliquors are e.g., oleic acid decyl ester, hydrated castor oil, light mineral oil, mineral oil, polyethylene glycol, sodium laurylsulfate.

Consistency enhancers are e.g., cetyl alcohol, cetyl ester wax, hydrated castor oil, microcrystalline waxes, non-ionic emulsifier waxes, beeswax, paraffin or stearyl alcohol.

Suitable hydrotropes are alcohols such as ethanol, isopropyl alcohol or polyols such as glycerin. Sorbents are materials that soak up oil from the water. Suitable examples include natural sorbents such as peat moss, sawdust, feathers, and anything else natural that contains carbon and synthetic sorbents such as polyethylene and nylon. Sorbents are used for tablet/capsule moisture-proofing by limited fluid sorbing (taking up of a liquid or a gas either by adsorption or by adsorption) in a dry state.

Opacifiers are substances that render the drinkable liquid opaque, if desired. They must have a refractive index substantially different from the solvent, in most cases here water. At the same time they should be inert to the other components of the composition. Suitable examples include titanium dioxide, talc, calcium carbonate behenic acid, cetyl alcohol, or mixtures thereof.

In another aspect of the invention the present application relates to a pharmaceutical composition comprising 5-amino-2,3-dihydro-1,4-phthalazinedione or one of its pharmaceutically acceptable salts and at least one glucocorticoid according to the disclosure for use in a formulation for oral administration in the prophylaxis or treatment of a disease usually treated with glucocorticoids. These pharmaceutical formulations suitable for oral dosage forms may be administered as separate units such as tablets, soft gelatin capsules, hard gelatin capsules, sugar-coated tablets or pills; powders or granulates; juices, syrups, drops, teas, solutions or suspensions in aqueous or non-aqueous liquids; edible foams or mousses; or in oil-in-water or water-in-oil in emulsions.

In oral dosage forms such as tablets or capsules the active agents can thus be combined with a non-toxic and pharmaceutically acceptable inert carrier such as ethanol, glycerol or water. Powders are produced by grinding the compound to a suitably tiny particle size and mixing them with a pharmaceutical carrier in a similar manner, e.g. an edible carbohydrate such as starch or mannitol. A flavor, preservative, dispersant or colorant can also be present.

Tablets are formulated by producing, granulating or dry-pressing a powder mixture, adding a lubricant and a disintegrants and pressing the mixture to a tablet. A powder mixture is produced by mixing a suitably ground compound with a diluent or a base as described before, and if applicable, with a binding agent such as carboxymethyl cellulose, an alginate, gelatin or polyvinyl pyrrolidone, a dissolution retardant, such as, for example, paraffin, an absorption accelerator, such as, for example, a quaternary salt, and/or an absorbent, such as, for example, bentonite, kaolin or dicalcium phosphate. The powder mixture can be granulated by wetting it with a binder, such as, for example, syrup, starch paste, acacia mucilage or solutions of cellulose or polymer materials and pressing it through a sieve. As an alternative to granulation, the powder mixture can be run through a tableting machine, giving lumps of non-uniform shape which are broken up to form granules. The granules can be lubricated by addition of stearic acid, a stearate salt, talc or mineral oil in order to prevent sticking to the tablet casting mold. The lubricated mixture is then pressed to give tablets. The compounds according to the invention can also be combined with a free-flowing inert excipient and then pressed directly to give tablets without carrying out the granulation or dry-pressing steps.

In another aspect of the invention a pharmaceutical composition comprising 5-amino-2,3-dihydro-1,4-phthalazinedione or one of its pharmaceutically acceptable salts and at least one glucocorticoid is provided in hard gelatin capsules. They are fabricated by producing a powder mixture as described before and filling it into shaped gelatin covers. Glidants and lubricants such as highly dispersed silica, talcum, magnesium stearate, calcium stearate or polyethylene glycol can be added to the powder mixture as solids. A disintegrant or solubilizer such as agar agar, calcium carbonate or sodium carbonate can be added likewise in order to improve the availability of the medication after intake of the capsule. Additionally, suitable binding agents and/or colorants can be added to the mixture, if desirable or necessary.

In another aspect of the invention a pharmaceutical composition comprising 5-amino-2,3-dihydro-1,4-phthalazinedione or one of its pharmaceutically acceptable salts and at least one glucocorticoid is included in soft gelatin capsules (SGC). SGCs are dissolved on their passage through the gastrointestinal tract. They consist mainly of gelatin enriched with variable amounts of plasticizers such as glycerol or sorbitan. The release rate depends on the specific formulation of the SGC carrier material. They are also suitable for a sustained release of the active agent. SGCs are particularly useful for the administration of poorly water-soluble active agents.

In another aspect of the invention a pharmaceutical composition comprising 5-amino-2,3-dihydro-1,4-phthalazinedione or one of its pharmaceutically acceptable salts and at least one glucocorticoid is included in chewable tablets or hard caramels. Herein the substance is integrated into the matrix of the tablets or caramels.

In another aspect of the invention the present application relates to a pharmaceutical composition comprising 5-amino-2,3-dihydro-1,4-phthalazinedione or one of its pharmaceutically acceptable salts and at least one glucocorticoid for use in the prophylaxis or treatment of a disease usually treated with glucocorticoids in a formulation for inhalatory administration.

For an effective prophylactic or therapeutic treatment of a pulmonary disease usually treated with glucocorticoids it is advantageous that the pharmaceutical composition comprising 5-amino-2,3-dihydro-1,4-phthalazinedione or one of its pharmaceutically acceptable salts and at least one glucocorticoid reaches the patient's alveoli. Therefore, the particle size must be sufficiently small to reach the lowest parts of the airways of the pulmonary tissue. The best inhalatory device class for inhalatory application of a pharmaceutically active agent are the so-called mesh nebulizers. In the scope of the present application practically all mesh nebulizers known in the art can be used, from rather simple single-use mesh nebulizers for cough and cold or for fancy purposes to sophisticated high-end mesh nebulizers for clinical or domestic treatment of serious diseases or conditions of the lower airways.

Suitable are all commercially available mesh nebulizers, jet nebulizers, ultrasonic nebulizers, dry powder inhalers and (pressurized) metered-dose inhalers.

Preferred are mesh nebulizers with a piezoelectric activation of the nebulization process, respectively vibrating mesh nebulizers.

Thus, in another aspect of the invention the present application relates to a pharmaceutical composition comprising 5-amino-2,3-dihydro-1,4-phthalazinedione or one of its pharmaceutically acceptable salts and at least one glucocorticoid for use in the prophylaxis or treatment of a pulmonary disease usually treated with glucocorticoids in a formulation for inhalatory administration, wherein the inhalatory administration is carried out by means of a vibrating mesh nebulizer.

Mesh nebulizers can be classified into two groups according to patient interaction: Continuous mode devices and trigger-activated devices. In continuous mode mesh nebulizers the nebulized aerosol is continuously released into the mouthpiece and the patient has to inhale the provided aerosol. In trigger-activated devices a defined amount of aerosol is released only upon an active and deep inspiratory breath. This way a far larger amount of active agent-containing aerosol is inhaled and reaches the lowest airways than with continuous mode devices. The latter lose a large amount of active agent-containing aerosol either to the surrounding or on the passage of the upper airways, as the aerosol release is not coupled to the respiratory cycle.

Therefore, trigger-activated mesh nebulizers are preferred, in particular vibrating mesh nebulizers.

Particularly preferred are trigger-activated mesh nebulizers with a piezoelectric activation of the nebulization process.

Preferred are the mesh nebulizer models PARI eFlow^{®}rapid, Philips Respironics I-neb, Philips InnoSpire Go, M-neb^{®} dose⁺ mesh nebulizer inhalation MN-300/8, Hcmed Deepro HCM-86C and HCM860, OMRON MicroAir U100, Aerogen^{®} Solo, KTMED NePlus NE-SM1, Vectura Fox, Vectura Bayer Breelib^{™}.

The most preferred vibrating mesh nebulizer models are high-end models such as PARI eFlow^{®}rapid, PARI Velox, Philips Respironics I-neb, M-neb^{®} dose⁺ mesh nebulizer inhalation MN-300/8, Aerogen^{®} Solo, Vectura Fox, Vectura Bayer Breelib^{™}.

In yet another aspect of the invention a pharmaceutical composition comprising 5-amino-2,3-dihydro-1,4-phthalazinedione or one of its pharmaceutically acceptable salts and at least one glucocorticoid for use in the prophylaxis or treatment of a pulmonary disease usually treated with glucocorticoids, wherein said pharmaceutical composition is provided as an additive to the ventilation air of a cardiopulmonary bypass device, a form of assisted ventilation. When a patient's condition in intensive care unit worsens, they often need to be ventilated in such a device for an indefinite period of time until their own respiration would allow for a sufficient oxygen supply. Good results have been achieved with an aerosol in a metered-dose inhaler combined with an inhalation chamber at the Y-piece. Alternatively, constant output mesh nebulizers or vibrating mesh nebulizers, ultrasonic or jet nebulizers may be used. Pros and cons of the different types are known by the expert in the field and are described in scientific literature.

In these cases, the pharmaceutical composition comprising 5-amino-2,3-dihydro-1,4-phthalazinedione or one of its pharmaceutically acceptable salts and at least one glucocorticoid can be added to the intubated ventilation air in solid form (dry powder) or in liquid form (in an aqueous solution or as a nebulized aerosol, as described before).

In yet another aspect of the invention the present application relates to a pharmaceutical composition comprising 5-amino-2,3-dihydro-1,4-phthalazinedione or one of its pharmaceutically acceptable salts and at least one glucocorticoid for use in the prophylaxis or treatment of a disease usually treated with glucocorticoids in a formulation for sublingual tablets. Sublingual drug delivery can be an alternative when compared to oral drug delivery as sublingually administered dosage forms bypass hepatic metabolism. A rapid onset of pharmacological effect is often desired for some drugs, especially those used in the treatment of acute disorders. Sublingual tablets disintegrate rapidly, and the small amount of saliva present is usually sufficient for achieving disintegration of the dosage form coupled with better dissolution and increased bioavailability.

The drug must be lipophilic enough to be able to partition through the lipid bilayer, but not so lipophilic such that once it is in the lipid bilayer, it will not partition out again. According to the diffusive model of absorption, the flux across the lipid bilayer is directly proportional to the concentration gradient. Therefore, lower salivary solubility results in lower absorption rates and vice versa. In general, a drug which has been formulated for sublingual should ideally have a molecular weight of less than 500 to facilitate its diffusion. The oral cavity has a narrow pH range which lies between 5.0 to 7.0. The inclusion of a suitable buffer during the formulation of an ionizable drug makes it possible to control the pH of aqueous saliva.

In order to avoid a possibly unpleasant taste or smell of the drug taste masking is needed. Sweeteners, flavors, and other taste-masking agents are essential components. Sugar-based excipients quickly dissolve in saliva and produce endothermic heat of dissolution. They create a pleasant feeling in the mouth and are most suitable for sublingual tablets along with other flavors.

Typical techniques for manufacturing sublingual tablets include direct compression, compression molding, freeze drying and hot melt extrusion (Khan et al. (2017) J Pharmaceut Res 16: 257-267).

In yet another aspect of the invention the present application relates to a pharmaceutical composition comprising 5-amino-2,3-dihydro-1,4-phthalazinedione or one of its pharmaceutically acceptable salts and at least one glucocorticoid for use in the prophylaxis or treatment of a disease usually treated with glucocorticoids in a liquid dosage form.

The present application discloses also the parenteral administration of pharmaceutical composition comprising 5-amino-2,3-dihydro-1,4-phthalazinedione or one of its pharmaceutically acceptable salts and at least one glucocorticoid in the prophylaxis or treatment of a disease usually treated with glucocorticoids in the form of intravenous injection, intraarterial injection or intraperitoneal injection.

These liquid dosage forms comprise solutions, suspensions and emulsions. Examples are water and water/propylene glycol solutions for parenteral injections, or the addition of a sweetener or opacifier for oral solutions, suspensions and emulsions.

These liquid dosage forms can be stored in vials, IV bags, ampoules, cartridges, and prefilled syringes. Suitable excipients include solubilizers, stabilizers, buffers, tonicity modifiers, bulking agents, viscosity enhancers/reducers, surfactants, chelating agents, and adjuvants.

In yet another aspect of the invention a pharmaceutical composition comprising 5-amino-2,3-dihydro-1,4-phthalazinedione or one of its pharmaceutically acceptable salts and at least one glucocorticoid for use in the prophylaxis or treatment of a disease usually treated with glucocorticoids is formulated as a lyophilizate. A lyophilizate can be reconstituted with water for injection or physiological saline or a water/ethanol solution and then be administered by injection.

Typical application forms for intravenous injections include infusion pumps, hypodermic needles, drip chambers, peripheral cannulae (peripheral venous catheters) and pressure bags.

In general, an aqueous solution or a physiological saline solution is preferred. In case of a poorly soluble pharmaceutical agent according to the invention also ethanol or ethanol/water mixtures can be used.

Further suitable liquid dosage forms include drops, eyedrops and eardrops.

The common formulation types used for nasal spray products are solutions, suspensions, and emulsions. Nasal spray formulations may be aqueous, hydroalcoholic, or nonaqueous-based. Depending on the type of system, the formulation will include a range of functional excipients, including solvents and cosolvents; mucoadhesive agents; pH buffers; antioxidants;
preservatives; osmolality and tonicity agents; penetration enhancers; suspending agents;
and surfactants. The choice of formulation type and the excipients selected will be driven by the solubility and stability of the pharmaceutical composition comprising 5-amino-2,3-dihydro-1,4-phthalazinedione or one of its pharmaceutically acceptable salts and at least one glucocorticoid, as well as the concentration needed to deliver an efficacious dose in a typical 100 µl spray (cf. Kulkarni and Shaw (2016) in: Essential Chemistry for Formulators of Semisolid and Liquid Dosages, Elsevier). The aforementioned Carragelose^{®} technique is used also for nasal sprays.

Nose drops are administered in a similar formulation but dropwise instead of a push on the dispenser.

The present application relates also to a pharmaceutical composition comprising 5-amino-2,3-dihydro-1,4-phthalazinedione or one of its pharmaceutically acceptable salts and at least one glucocorticoid, wherein the administration of this pharmaceutical composition is carried out by means of eye drops.

Eye drops are mostly aqueous solutions containing a pharmaceutically active agent. The pH is usually adjusted to 7.1 to 7.5. Common buffers for eye drops are boric acid and monobasic sodium phosphate. The tonicity should be adjusted by 0.9 % saline (or another isotonizing agent such as potassium nitrate, boric acid, sodium acetate, sodium acetate phosphate buffer or mannitol) to an osmotic pressure isotonic to the cornea epithelium (225 - 430 mosm/kg). Suitable preservatives include thiomersal, organic mercury compounds such as phenyl mercury, benzalkonium chloride, chlorhexidine, and benzylic alcohol. For prolonging the contact time viscosity-increasing substances (thickening agents) such as cellulose derivatives (hypromellose, methylcellulose, hydroxypropyl methylcellulose), hyaluronic acid, cellulose acetate phthalate, polyethylene glycol, polyvinyl alcohols or poloxamers can be added. Wetting agents or surfactants such as benzalkonium chloride, polysorbate 20, polysorbate 80, dioctyl sodium sulphosuccinate can be included. Some amino acids, alone or in combination with sodium hyaluronate may be helpful in promoting tissue reconstitution, if needed. Suitable amino acids are glycine, leucine, lysine and proline.

In yet another embodiment of the invention a pharmaceutical composition comprising 5-amino-2,3-dihydro-1,4-phthalazinedione or one of its pharmaceutically acceptable salts and at least one glucocorticoid according to the disclosure is provided as a topical application form, such as creams, emulsions, lotions, gels, hydrogels, pastes, powders, ointments, liniment, films, liposomes, dermal patches, transdermal patches, transdermal sprays or suspensions.

Specifically for the production of suppository waxes with a low melting point a mixture of fatty acid glycerides such as cocoa butter are first melted, then the active substance is or the active substances are homogenously dispersed under stirring or other mixing methods. The molten homogeneous mixture is then transferred to suitable molds and cooled down until solidification. All the aforementioned substances and classes of substances can be used as excipients according to the invention, alone or in any conceivable combination thereof.

The present application refers likewise to a composition for use in the prophylaxis and/or treatment of conditions or diseases usually treated with glucocorticoids, wherein said composition contains at least one of 5-amino-2,3-dihydro-1,4-phthalazinedione or any of aforementioned molecular variants of this compound, at least one excipient and at least one further non-steroidal active agent. Such a pharmaceutical composition optionally further comprises at least one glucocorticoid.

The at least one further active agent can be selected from a group comprising non-steroidal anti-inflammatory drugs; immunomodulators; immunostimulatory agents; immunosuppressive agents; antibiotics; anti-infective agents; antiviral agents; antifungal agents; antiprotozoal agents; anthelmintics; analgesics; local anesthetics; anticoagulants; antiplatelet drugs; muscle relaxants; bronchodilators; pulmonary vasodilators; mucolytic agents; pulmonary surfactants; antioxidants; ENaC-activating agents; HMG-CoA reductase inhibitors; AT₁ receptor antagonists; tonic agents; and anabolic agents.

Suitable examples for such non-steroidal anti-inflammatory drugs (NSAIDs) comprise acetylsalicylic acid, salicylic acid and salicylates, acetaminophen (paracetamol), salsalate, diflunisal, ibuprofen, dexibuprofen, naproxen, fenoprofen, ketoprofen, dexketoprofen, flurbiprofen, oxaprozin, loxoprofen, indomethacin, tolmetin, sulindac, etodolac, ketorolac, diclofenac, aceclofenac, nabumetone, piroxicam, meloxicam, tenoxicam, droxicam, lornoxicam, isoxicam, phenylbutazone, mefenamic acid, meclofenamic acid, flufenamic acid, tolfenamic acid, celexoxib, rofecoxib, valdecoxib, parecoxib, lumiracoxib, etoricoxib, firocoxib, nimesulide, clonixin, licofelone, H-harpagide, flunixin, tiaprofenic acid.

Suitable examples for such immunomodulators (IMIDs) comprise thalidomide, lenalidomide, pomalidomide and apremilast.

Suitable examples for such antiviral drugs comprise ancriviroc, aplaviroc, cenicriviroc, enfuvirtide, maraviroc, vicriviroc, amantadine, rimantadine, pleconaril, idoxuridine, aciclovir, brivudine, famciclovir, penciclovir, sorivudine, valaciclovir, cidofovir, ganciclovir, valganciclovir, sofosbusvir, foscarnet, ribavirine, taribavirine, filibuvir, nesbuvir, tegobuvir, fosdevirine, favipiravir, merimepodib, asunaprevir, balapiravir, boceprivir, ciluprevir, danoprevir, daclatasvir, narlaprevir, telaprevir, simeprevir, vanipevir, rupintrivir, fomivirsen, amenamevir, alisporivir, bevirimate, letermovir, laninamavir, oseltamivir, peramivir, zanamivir, remdesivir. Suitable examples for such immunostimulatory agents comprise interferons (α-, β-, γ-, τ-interferon), interleukins, CSF, PDGF, EGF, IGF, THF, levamisole, dimepranole, inosine.

Suitable examples for such immunosuppressive drugs comprise cytostatics such as alkylating agents (such as cyclophosphamide), antimetabolites such as methotrexate, azathioprine, mercaptopurine, fluorouracil, leflunomide, protein synthesis inhibitors and certain antibiotics such as dactinomycin, anthracyclines, mitomycin C, bleomycin and mithramycin, intercalating agents such as mitoxantrone; antibodies such as muromonab-CD3, rituximab, ustekinumab, alemtuzumab, natalizumab, basiliximab and daclizumab; drugs acting on immunophilins such as ciclosporin, tacrolimus and sirolimus; and non-classified immunosuppressive drugs such as β-interferon, γ-interferon, opioids, TNF binding proteins such as infliximab, etanercept, adalimumab; or curcumin, catechins, mycophenolic acid, fingolimod, myriocin and fumaric acid dimethyl esters.

Suitable examples for such antibiotics comprise imipenem, meropenem, ertapenem, cephalosporins, aztreonam, penicillins such as penicillin G and penicillin V, piperacillin, mezlocillin, ampicillin, amoxicillin, flucloxacillin, methicillin, oxacillin, clavulanic acid, sulbactam, tazobactam, sultamicillin, fosfomycin, teicoplanin, vancomycin, bacitracin, colistin, gramicidin, polymyxin B, tyrothricin, teixobactin, fosmidomycin, amikacin, gentamicin, kanamycin, neomycin, netilmicin, streptomycin, tobramycin, chloramphenicol, fusidic acid, cethromycin, narbomycin, telithromycin, clindamycin, lincomycin, daptomycin, dalfopristin, quinupristin, azithromycin, clarithromycin, erythromycin, roxithromycin, linezolid, doxycycline, minocycline, tetracycline, oxytetracycline, tigecycline, norfloxacin, enoxacin, ciprofloxacin, ofloxacin, levofloxacin, moxifloxacin, metronidazole, tinidazole, aminocumarine, sulfadiazine, sulfadoxin, sulfamethoxazole, sulfasalazine, pyrimethamine, trimethoprim, rifampicin. Anti-infective agents is a generic term for compounds that are useful in the treatment of bacterial, viral, fungal, protozoal and worm infections and comprises antibiotics, antiviral agents, antimycotics agents and anthelminthic agents.

Suitable examples for such muscle relaxants comprise tercuronium, 1-ethylcarbamoyl-3-(3-trifluoromethylphenyl)pyrrolidine, metaxalone, methocarbamol, meprobamate, baclofen, carisoprodol, chlorzoxanzone, cyclobenzaprine, dantrolene, diazepam, orphenadrine, quinine, rocuronium, succinylcholine, decamethonium, pancuronium, veruronium, rapacuronium, dacuronium, duador, malouetine, dipyrandium, pipercuronium, chandonium, HS-342, atracurium, mivacurium, doxacurium, d-tubocurarine, dimethyltubocurarine, gallamine, alcuronium, anatruxonium, diadonium, fazadinium, tropeinium, cisatrucurium.

Suitable examples for such antifungal drugs comprise abafungin, amphotericin B, candicidin, filipin, hamycin, natamycin, nystatin, rimocidin, bifonazole, butoconazole, clomitrazole, econazole, fenticonazole, isoconazole, ketoconazole, luliconazole, miconazole, omoconazole, oxiconazole, sertaconazole, sulconazole, tioconazole, albaconazole, efinaconazole, epoxiconazole, fluconazole, isavuconazole, itraconazole, posaconazole, propiconazole, ravuconazole, terconazole, voriconazole, amorolfin, butenafine, nafitifine, terbinafine, anidulafungin, caspofungin, micafungin, benzoic acid, ciclopirox, flucytosine, griseofulvin, haloprogin, tolnaftate, undecylenic acid, crystal violet, balsam of Peru.

Suitable examples for such antiprotozoal drugs comprise metronidazole, tinidazole, ornidazole, atovaquone, clioquinol, chlorquinaldol, emetin, pentamidine isethionate, eflornithine, nitrofural, halofuginone, miltefosine, chloroquine, hydroxychloroquine, mepacrine, primaquine, amodiaquine, pamaquine, piperaquine, proguanil, cyclohunailembonate, quinine, mefloquine, pyrimethamine, artmether, artemisinine, artesunate, dihydroartemisinine, halofantrine, lumefantrine, sulfadoxine.

Suitable examples for such anthelmintics comprise mebendazole, praziquantel, albendazole, diethylcarbamazine, flubendazole, ivermectin, levamisole, metrifonate, niclosamide, oxyclozanide, oxamniquine, oxantel, piperazine, pyrantel, pyrantel pamoate, monopantel, derquantel, pelletierine sulphate, pyrvinium, thiabendazole, fenbendazole, triclabendazole, abamectin, suramine, emodepside, pyrvinium embonate, aminoacetonitrile.

Suitable examples for further antiparasitic drugs comprise meglumine antimoniate, benznidazole, sodium stibogluconate, fumagillin, halofantrine, melarsoprol, nifurtimox, nitazoxanide, permethrin, lindane, malathion, carbaryl, pyrethrum, phenothrin, bio-allethrin, imidacloprid, moxidectin, nitenpyram, fipronil, pyriprol, selamectin, dimpylate, spinosad, indoxacarb, methoprene, pyriproxyfen, lufenuron, neem oil, citronella oil, clove oil, peppermint oil, eucalyptus oil.

Suitable examples for such local anesthetics comprise lidocaine, lignocaine, menthol, articaine, bupivacaine, ropivacaine, benzocaine, chloroprocaine, cocaine, cyclomethycaine, dimetociane, larocaine, piperocaine, propoxycaine, procaine, novocaine, proparacaine, tetracaine, amethocaine, cinchocaine, dibucaine, etidocaine, levobupivacaine, meplavacaine, prilocaine, trimecaine, saxitoxin, neosaxitoxin, tetrodotoxin, eugenol.

Suitable examples for analgesics comprise the NSAIDs listed above; opioid analgesics such as morphine, fentanyl, methadone, oxycodon, carfentanyl, dihydroetorphin, ohmefentanyl, etorphin, sufentanil, remifentanil, alfentanil, buprenorphine, hydromorphone, levomethadone, hydrocodone, piritramide, nalbuphine, tapentadol, pentazocin, dihydrocodeine, codeine, pethidine, tramadol, tilidine, meptazinol, naloxone, naltrexone, diprenorphine, loperamide, apomorphine; epibatidine; scopolamine; ziconitide; cannabinoids such as tetrahydrocannabinol, cannabidiol, marinol; flupirtine; ketamine and local anesthetics listed above.

Suitable examples for such anticoagulants comprise heparins, coumarins such as phenprocoumon (marcumar) and warfarin, apixaban, rivaroxaban, edoxaban, dabigatran, betrixaban, ximelagatran, hirudine, lepirudine, bivalirudine, citrate, EDTA, fondaparinux, idraparinux, idrabiotaparinux, argatroban, otamixaban.

Suitable examples for such antiplatelet agents comprise abciximab, acetylsalicylic acid, dipyridamole, clopidogrel, eptifibatide, ilomedin, prostacyclin, prasugrel, ticagrelor, ticlopidine, tirofiban.

Suitable bronchodilators such as beta-2 adrenergic receptor agonists comprise short-acting beta-2 agonists (SABAs) such as salbutamol, albuterol, bitolterol, fenoterol, isoprenaline, levosalbutamol, levalbuterol, orciprenaline, pirbuterol, procaterol, ritodrine and terbutaline; long-acting beta-2 agonists (LABAs) such as arformoterol, bambuterol, clenbuterol, formoterol and salmeterol; ultra-long-acting beta-2 agonists such as abediterol, carmoterol, indacaterol, olodaterol and vilanterol, alone or combined with umeclidinium bromide and/or fluticasone furoate; beta-2 agonists with unknown time of action such as isoxsuprine, mabuterol or zilpaterol.

Suitable muscarinic anticholinergics (bronchodilating M3 receptor antagonists) comprise ipratropium bromide, tiotropium bromide, oxitropium bromide, glycopyrronium bromide, aclidinium bromide, umeclidinium bromide, atropine, hyoscyamine, aclidinium bromide, 4-DAMP, darifenacin, DAU-5884, HL-031, HL-120, J-104, J-129, procyclidine, oxybutynin, tolterodine and zamifenacin.

Further bronchodilators comprise epinephrine, ephedrine, theophylline and TSG12.

A potent pulmonary vasodilator is nitric oxide. Further suitable pulmonary vasodilators are prostacyclin (prostaglandin PGI2) analogues such as iloprost, epoprostenol and treprostinil. Suitable mucolytic agents comprise N-acetylcysteine (NAC), ambroxol, bromhexine, carbocisteine, erdosteine, mecysteine and dornase alfa.

Suitable pulmonary surfactants comprise synthetic compositions such as Colfosceril palmitate, Pumactant, KL-4, Venticute and Lucinactant as well as animal-derived surfactants such as Beractant, Calfactant and Poractant alfa.

A potent antioxidant is inhaled carbon monoxide (CO).

Suitable ENaC (epithelium sodium channel) activating peptides comprise AP301 and S3969.

Suitable HMG-CoA reductase inhibitors (statins) comprise atorvastatin, alone or in combination with amlodipine and/or perindopril, cerivastatin, fluvastatin, lovastatin, alone or in combination with niacin, mevastatin, pitavastatin, pravastatin, rosuvastatin, alone or in combination with ezetimibe, simvastatin, alone or in combination with ezetimibe or niacin.

Suitable AT₁ antagonists (angiotensin II receptor blockers; sartans) comprise losartan, valsartan, candesartan, telmisartan, irbesatan, olmesartan, eprosartan, fimasartan, azilsartan, milfasartan, pomisartan, pratosartan, ripisartan, tasosartan, saprosartan and EXP 3174.

Tonic agents is a generic term that refers to substances that invigorate, tone or restore the body and its physiological functions. They may be of herbal or animal origin.

Anabolic drugs may be useful for the anabolism and strengthening of the cellular collagen scaffold. However, there has been a broad abuse of these substances for doping in sports and for body building. Therefore, their use in a combination is only encouraged if no national legal bans of these anabolic compounds are infracted.

Application forms of the compositions according to the invention as outlined above comprise but are not limited to oral, parenteral, intravenous, intraarterial, by inhalation, by intubation, intramuscular, topical, transdermal, subcutaneous, intradermal, transmucosal, sublingual, buccal, conjunctival, intravaginal, rectal or nasal administration.

Depending on target tissue systemic applications not always yield the desired results. To avoid the administration of increased dosages an alternative topical route of administration may be desirable in these cases.

It was found that the injection of an active substance directly into the affected tissue, the surrounding tissue or body fluid may lead to improved results in respect of drug effect, safety and/or pharmacokinetics.

Therefore, the present application likewise refers also to the use of compositions according to the invention, wherein a parenteral application is carried out by injection of said composition into the affected tissue, the surrounding tissue or surrounding body fluid.

Suitable examples comprise but are not limited to intraperitoneal, intraosseal, periosseal, intraarticular, periarticular, epidural, peridural, intrathecal, intravitreal, intravesical and intrapericardial injection.

If the affected tissue is part of the skin or located close to the skin surface it may be preferable to use a topical application on the skin for the same reasons as laid out before.

Therefore, the present application refers also to the use of compositions according to the invention, wherein a topical application is carried out by means of a cream, emulsion, lotion, gel, hydrogel, paste, powder, ointment, liniment, film, liposomes, dermal patch, transdermal patch, transdermal spray or suspension.

Improved results in such topical applications in respect of efficacy, safety and/or pharmacokinetics can be achieved when a permeation enhancer is included in the topical dosage form. Suitable permeation enhancers have been listed above.

### EXAMPLES

### Example 1 - Effects of 5-amino-2,3-dihydro-1,4-phthalazinedione sodium salt and dexamethasone on LPS stimulated RAW264.7 cells:

In order to test whether a combination of dexamethasone and 5-amino-2,3-dihydro-1,4-phthalazinedione sodium salt shows a synergistic effect and/or allows for a booster effect, the following experiments were carried out with 5-amino-2,3-dihydro-1,4-phthalazinedione sodium salt corresponding to Form I as defined earlier in the description. Raw264.7 cells (leukemia transformed monocytic-macrophage cell line derived from mouse ascitic fluid) were grown in Dulbecco's Modified Eagle's Medium enriched with glutamine and fetal calf serum and maintained at 37°C and 5% CO₂.

RAW264.7 cells were pre-incubated with 5-amino-2,3-dihydro-1,4-phthalazinedione sodium salt (dose range 0.025 mM - 1 mM) and the following concentrations of dexamethasone (1, 0.1, and 0.01 µM, as well as sub-threshold concentrations of serial two-fold dilutions spanning a range from 0.01 - 0.0003 µM) for 1 h before LPS (lipopolysaccharides) stimulation (100 ng/ml). After 24 hours interleukin 6 (IL-6) levels were determined in supernatants by an ELISA assay and compared with supernatants from cells treated with 5-amino-2,3-dihydro-1,4-phthalazinedione sodium salt or dexamethasone alone, respectively.

Two experimental parts were performed. In part 1 of the experiment dexamethasone was used at three ten-fold scalar concentrations (1, 0.1 and 0.01 µM) and incubated with two-fold scalar concentrations of 5-amino-2,3-dihydro-1,4-phthalazinedione sodium salt (1 - 0.025 mM range). A complete summary of the percentage reduction in IL-6 production for these combinations of 5-amino-2,3-dihydro-1,4-phthalazinedione sodium salt and dexamethasone is presented in Table 1. Part 2 of the experiment has been performed to further demonstrate the effect of the combination of 5-amino-2,3-dihydro-1,4-phthalazinedione sodium salt and dexamethasone on IL-6 levels when suboptimal doses of dexamethasone are used. Hence, in experimental part 2 six serial two-fold dilutions of dexamethasone starting from 0.01µM as the highest concentration were administered together with 5-amino-2,3-dihydro-1,4-phthalazinedione sodium salt. A complete summary of the percentage reduction in IL-6 production for these further combinations of 5-amino-2,3-dihydro-1,4-phthalazinedione sodium salt and dexamethasone is also presented in Table 1.

| **Table 1. Percentage inhibition of IL-6 production for each combination of 5-amino-2,3-dihydro-1,4-phthalazinedione sodium salt and dexamethasone and for 5-amino-2,3-dihydro-1,4-phthalazinedione sodium salt and dexamethasone alone.** Values marked with * display supraadditivity based on simple arithmetic sums. Values marked with ° were not applicable to determine supraadditivity via simple arithmetic sums. Slightly negative values indicating there was no inhibition at all are displayed as zero. | | | | | | | | |
|---|---|---|---|---|---|---|---|---|
| | | 5-amino-2,3-dihydro-1,4-phthalazinedione sodium salt (mM) | | | | | | |
| Dexamethasone (µM) | | 1 | 0.5 | 0.25 | 0.1 | 0.05 | 0.025 | 0 |
| Exp Part 1 | 1 | 99.00° | 94.09° | 90.60° | 89.18° | 93.05° | 91.55° | 87.00 |
| | 0.1 | 94.14° | 88.06° | 90.64° | 82.00° | 75.71 | 71.77 | 63.81 |
| | 0.01 | 92.37° | 70.57 | 69.03 | 69.41 | 65.33* | 59.37* | 34.66 |
| | 0 | 72.75 | 56.70 | 53.95 | 44.85 | 25.77 | 17.03 | 0 |
| Exp Part 2 | 0.01 | 86.12° | 76.83 | 60.41* | 50.12* | 45.35* | 47.40* | 36.27 |
| | 0.005 | 84.50 | 71.12 | 59.47* | 47.49* | 30.96* | 32.08 | 30.57 |
| | 0.0025 | 72.18* | 56.33* | 42.07* | 20.28* | 9.61* | 11.69* | 4.16 |
| | 0.0012 | 66.78* | 57.72* | 27.67* | 22.99* | 3.64* | 7.16* | 0 |
| | 0.0006 | 62.76 | 48.38 | 37.66* | 12.76* | 0 | 0 | 4.07 |
| | 0.0003 | 54.45 | 52.45 | 31.29* | 15.74* | 0.34* | 0 | 1.57 |
| | 0 | 67.30 | 47.52 | 13.85 | 0 | 0 | 1.57 | 0 |

### A - Synergistic effects

The general synergistic effect of combinations of dexamethasone and 5-amino-2,3-dihydro-1,4-phthalazinedione sodium salt could be proven for both 5-amino-2,3-dihydro-1,4-phthalazinedione sodium salt as add on to dexamethasone (see table 2) and vice versa (not shown) in both experimental parts as described above.

| **Table 2. Synergistic inhibition of IL-6 production of** 5-amino-2,3-dihydro-1,4-phthalazinedione sodium salt **when added to dexamethasone** displayed as the absolute difference between the percentual IL-6 inhibitions by combinations of 5-amino-2,3-dihydro-1,4-phthalazinedione sodium salt and dexamethasone compared to IL-6 inhibition by dexamethasone alone. Values in brackets show the relative (percentual) improvements of the combinations. Combinations which showed an antagonistic effect are marked with an X. | | | | | | | |
|---|---|---|---|---|---|---|---|
| | | 5-amino-2,3-dihydro-1,4-phthalazinedione sodium salt (mM) | | | | | |
| Dexamethasone (µM) | | 1 | 0.5 | 0.25 | 0.1 | 0.05 | 0.025 |
| Exp Part 1 | 1 | 12.0 (12.1) | 7.1 (7.5) | 3.6 (4.0) | 2.2 (2.4) | 6.0 (6.5) | 4.5 (5.0) |
| | 0.1 | 30.3 (32.2) | 24.2 (27.5) | 26.5 (29.4) | 18.2 (22.2) | 11.9 (15.7) | 8.0 (11.1) |
| | 0.01 | 57.7 (62.5) | 35.9 (50.9) | 34.4 (49.8) | 34.8 (50.1) | 30.7 (46.9) | 24.7 (41.6) |
| Exp Part 2 | 0.01 | 49.8 (57.9) | 40.6 (52.8) | 24.1 (40.0) | 13.8 (27.6) | 9.1 (20.0) | 11.1 (23.5) |
| | 0.005 | 53.9 (63.8) | 40.5 (57.0) | 28.9 (48.06) | 16.9 (35.6) | 0.4 (1.3) | 1.5 (4.7) |
| | 0.0025 | 68.0 (94.2) | 52.2 (92.6) | 37.9 (90.1) | 16.1 (79.5) | 5.4 (56.7) | 7.5 (64.4) |
| | 0.0012 | 83.5 (125.0) | 74.4 (128.9) | 44.4 (160.3) | 39.7 (172.6) | 20.3 (558.1) | 23.8 (333.3) |
| | 0.0006 | 58.7 (93.5) | 44.3 (91.6) | 33.6 (89.2) | 8.7 (68.1) | X | X |
| | 0.0003 | 53.6 (98.4) | 51.6 (98.3) | 30.4 (97.2) | 14.9 (94.4) | X | X |

In experimental part 1 all combinations performed showed a synergistic effect in this respect. The effect was more pronounced in combinations where 5-amino-2,3-dihydro-1,4-phthalazinedione sodium salt was added to lower doses of dexamethasone. In experimental part 2 - where 5-amino-2,3-dihydro-1,4-phthalazinedione sodium salt was added to suboptimal doses of dexamethasone - apart from four low dose / low dose combinations all other combinations performed showed a synergistic effect in this respect, as well. The effect was very pronounced in most of the combinations. The best synergistic effect was received when 5-amino-2,3-dihydro-1,4-phthalazinedione sodium salt in any concentration was added to 0.0012 µM dexamethasone. When adding 0.05 mM 5-amino-2,3-dihydro-1,4-phthalazinedione sodium salt to 0.0012 µM dexamethasone the effectiveness of the combination was even more than 5-fold higher than expected by pure additivity. These results confirmed the synergism between 5-amino-2,3-dihydro-1,4-phthalazinedione sodium salt and dexamethasone on the production of IL-6 from Raw264.7 cells. The synergism found works in both directions: Dexamethasone can synergistically improve the effect of 5-amino-2,3-dihydro-1,4-phthalazinedione sodium salt and 5-amino-2,3-dihydro-1,4-phthalazinedione sodium salt can synergistically improve the effect of dexamethasone.

### B - Supraadditive synergistic effects

A cumulative effect greater than expected by the combination of two individual effects is regarded as a supraadditive effect. Based on simple arithmetic sums in experimental part 1 such a supraadditive effect on IL-6 levels was observed for the combination of 5-amino-2,3-dihydro-1,4-phthalazinedione sodium salt and dexamethasone at the lowest concentrations used. Indeed, while 0.025 mM 5-amino-2,3-dihydro-1,4-phthalazinedione sodium salt and 0.01 µM dexamethasone inhibited IL-6 production by 17% and 34.7%, respectively, when administered as single drugs, the association reached an inhibitory effect of 59.4%. Similarly, 0.05 mM 5-amino-2,3-dihydro-1,4-phthalazinedione sodium salt inhibited IL-6 production by 25.8% and association with 0.01 µM dexamethasone reached an inhibitory effect of 65.3% (Table 1). In experimental part 2 - where subthreshold doses of dexamethasone were used - the great majority of combinations showed such a supraadditive effect. In Table 1 values that show supraadditivity according to this method are marked with an asterisk. Since an inhibition of more than 100 % is not possible, combinations with an expected sum higher than 100, which is true for most of the results gathered from experimental part 1, are not applicable for this method.

To allow the interpretation of all combinations investigated, including those that theoretically would sum up to more than 100 %, the fractional product method was applied to these data as well. Table 3 displays the difference between expected and measured percentage inhibition of IL-6 production for each combination of 5-amino-2,3-dihydro-1,4-phthalazinedione sodium salt and dexamethasone following this method.

It could be shown that almost all combinations of both experimental part 1, including a high number of non-interpretable combinations with the arithmetic sum method, and experimental part 2 are supraadditive.

| **Table 3. Difference between expected (based on fractional products method) and measured percentage inhibition of IL-6 production for each combination of** 5-amino-2,3-dihydro-1,4-phthalazinedione sodium salt **and dexamethasone**. Positive values indicate supraadditivity. | | | | | | | |
|---|---|---|---|---|---|---|---|
| | | 5-amino-2,3-dihydro-1,4-phthalazinedione sodium salt (mM) | | | | | |
| Dexamethasone (µM) | | 1 | 0.5 | 0.25 | 0.1 | 0.05 | 0.025 |
| Exp Part 1 | 1 | 2.5 | -0.3 | -3.4 | -3.7 | 2.7 | 2.3 |
| | 0.1 | 4.0 | 3.7 | 7.0 | 2.0 | 2.6 | 1.8 |
| | 0.01 | 10.2 | -1.1 | -0.9 | 5.5 | 13.8 | 13.6 |
| Exp Part 2 | 0.01 | 7.0 | 10.3 | 15.3 | 15.4 | 12.2 | 10.1 |
| | 0.005 | 7.2 | 7.6 | 19.3 | 18.6 | 3.7 | 0.4 |
| | 0.0025 | 3.5 | 6.6 | 24.6 | 18.4 | 10.1 | 6.0 |
| | 0.0012 | 4.9 | 19.0 | 28.2 | 42.5 | 26.0 | 22.0 |
| | 0.0006 | -5.9 | -1.3 | 20.3 | 11.0 | -7.0 | -6.5 |
| | 0.0003 | -13.1 | 4.5 | 16.7 | 17.3 | 4.2 | -5.1 |

Further, the Combination Index (CI) has been calculated for all combinations (non-constant ratio combination) received in Experiment 1 and 2 using the specialized software CompuSyn.

In experimental part 1 in all combinations investigated the CI values were smaller than 1 (Figure 1). Thus, according to this parameter, all combinations tested in experimental part 1 must be regarded as supraadditive. In experimental part 2 the CI value was again smaller than 1 in most combinations investigated apart from 3 exemptions only (0.0003µgDEX/0.025mg 5-amino-2,3-dihydro-1,4-phthalazinedione sodium salt, 0.0006µgDEX/0.025mg 5-amino-2,3-dihydro-1,4-phthalazinedione sodium salt, and 0.0006µgDEX/0.05mg 5-amino-2,3-dihydro-1,4-phthalazinedione sodium salt (Figure 2).

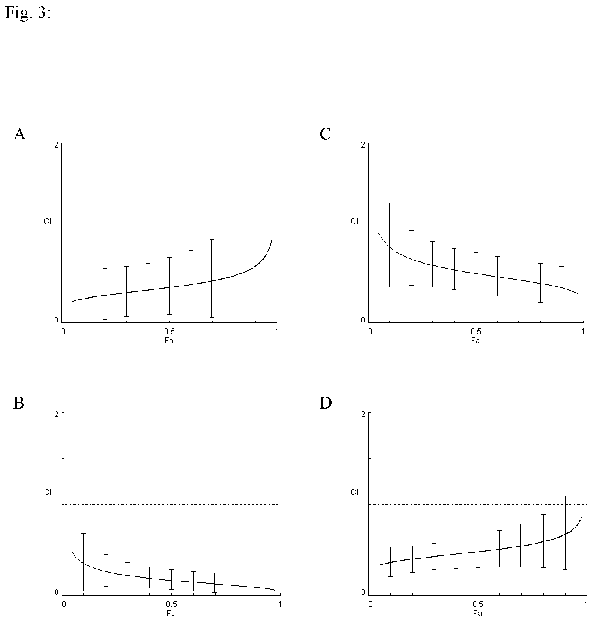

Results from both experimental parts allowed to calculate the CI for various fixed-ratio combinations of dexamethasone and 5-amino-2,3-dihydro-1,4-phthalazinedione sodium salt (4:1, 2:1, 1:1. 1.2.5, 1:5, and 1:10 in experimental part 1 and 1:5, 1:10, 1:20, 1:40, 1:50, 1:100, 1:200, 1:400, 1:800 and 1: 1600 in experimental part 2). Such a fixed ratio approach is based on different doses/concentrations of the active ingredients whereas the ratio between the active ingredients stays fixed. Based on the data received the CI is calculated along the increasing fractual effect. Although calculations for single parts of the curves sometimes result in a CI higher than 1, in most of the cases again clear supraadditivity is given. In other words, the FaCI plots of all fixed ratio combinations of both experimental parts were either completely or at least to their much greater part in the supraadditive area of the plot. Exemplarily some of these fixed ratio curves from Experiment 2 are shown in Figure 3.

### Example 2: Weight loss in mice treated with dexamethasone

It is a well-known phenomenon that study animals, in particular mice and rats, experience slower weight gain or even weight loss when they are administered glucocorticoids. This effect is induced and occurs independently from other conditions investigated in respective trials.

In a pilot study of collagen induced arthritis (CIA) of rheumatoid arthritis in mice applicant found that both arithmetic mean and median of the body weight at the end of the trial (day 45) was higher in groups that received a combination of dexamethasone and 5-amino-2,3-dihydro-1,4-phthalazinedione sodium salt compared to the groups that were administered dexamethasone alone (see table 4).

Hence there is a clear trend showing that the additional application of 5-amino-2,3-dihydrophthalazine-1,4-dione sodium salt to a given amount of dexamethasone helps to prevent side effects induced by the glucocorticoid.

In this trial arthritis was induced by subcutaneous injection of 100 µg chicken collagen type UU in Freund's adjuvant on day 0 and a respective boost using 50 µg on day 21. Test items (dexamethasone, 5-amino-2,3-dihydro-1,4-phthalazinedione sodium salt) were administered daily intraperitoneally after onset of disease when an individual disease score higher than 2 was reached. Scoring was done using macroscopic evaluation of the four limbs ranging from 0 to 15 wherein 1 point is given for each swollen or red toe 1 point for a swollen or red mid foot digit or knuckle, 5 points for a swollen ankle, resulting in a maximum total score of 60 for each mouse.

| **Table 4: Body weight (g) of mice on day 45 (end of trial) in a pilot study on rheumatoid arthritis.** Shown are arithmetic mean ± standard deviation. Values in brackets are median and sample size. | | | | | |
|---|---|---|---|---|---|
| Body Weight (g) | | Dexamethasone (mg/kg/day) | | | |
| | | 0 | 0.05 | 0.1 | 0.3 |
| 5-amino-2,3-dihydro-1,4-phthalazinedione sodium salt (mg/kg/day) | 0 | 22.37 ± 2.23 (22.2, n = 11) | 20.8 ± 1.18 (20.7, n = 9) | 20.72 ± 1.57 (21, n = 11) | 20.10 ± 1.61 (20, n = 12) |
| | 1 | 22.8 ± 1.68 (23.6, n =11) | 21.38 ± 1.46 (21.7, n = 10) | 21.05 ± 1.41 (21.3, n = 10) | |

### Example 3: Case report rheumatoid arthritis

A 53 years-old man was diagnosed with rheumatoid arthritis a few months after experiencing first symptoms. Due to a known NSAID (non-steroid anti-inflammatory drug) hypersensitivity treatment directly started with the administration of prednisone. Therefore, he was dosed with 10mg prednisone retard tablets once daily before going to bed. Both pain and inflammation improved within the next months; however, they did not completely disappear, and he also started to gain weight. After 8 months of glucocorticoid therapy 5-amino-2,3-dihydro-1,4-phthalazinedione sodium salt was added to the ongoing therapy, starting with the oral administration of 50 mg twice daily. 10 days after starting the combinatory treatment the patient felt much better, there was nearly no more pain and inflammation improved dramatically. 4 to 12 weeks after starting the combinatory treatment the prednisone dose was stepwise reduced to 5, 2 and 1 mg per day, respectively. The dose of 5-amino-2,3-dihydro-1,4-phthalazinedione sodium salt was gradually increased from 100 mg per day to 150, and 200 mg per day up to a maintenance dose of 250 mg per day, which turned out to be a good dose for long-term treatment.

### Example 4: Dosage scheme for Crohn's disease

The glucocorticoid treatment of a patient experiencing acute Crohn's disease being initially administered 60 mg of prednisolone per day which is then within the next 8 to 12 weeks stepwise lowered to a maintenance dose of 5 to 10 mg per day which should then be administered for about 3 months is replaced by a fixed ratio scheme.

According to the results presented in Example 1 any feasible ratio would be applicable. However, considering that dexamethasone has about 6-times the potency of prednisolone and that due to compliance there are practical limitations in the amount of 5-amino-2,3-dihydro-1,4-phthalazinedione sodium salt that can orally be administered to a patient for best results the following fixed dose ratios are particularly useful:
Possible pairs of prednisolone and 5-amino-2,3-dihydro-1,4-phthalazinedione sodium salt for the initial daily dose are for example 60 mg / 400 mg, 40 mg / 800 mg and 20 mg / 1200 mg.

The respective maintenance doses are 4 mg / 400 mg, 2 mg / 200 mg and 1 mg / 100 mg.

### Fig. 1: FaCI non-constant ratio combination - Example 1 - Experimental Part 1

The X coordinate displays the fractual effect (Fa), i.e., a value between 0 and 1 for the relative inhibition of IL-6 in LPS stimulated RAW264.7 cells, wherein 1 means a 100% inhibition and 0 means no inhibition. The Y coordinate displays the Combination Index as calculated via CompuSyn Software, wherein the value 1 shows additivity, values smaller than 1 show supraadditivity and values above 1 show subadditivity or antagonism.

Shown are all dose combinations of 5-amino-2,3-dihydro-1,4-phthalazinedione sodium salt and dexamethasone as investigated.

All CI values were below 1 and thus show a supraadditive effect.

### Fig. 2: FaCI non-constant ratio combination - Example 1 - Experimental Part 2

The X coordinate displays the fractual effect (Fa), i.e. a value between 0 and 1 for the relative inhibition of IL-6 in LPS stimulated RAW264.7 cells, wherein 1 means a 100% inhibition and 0 means no inhibition. The Y coordinate displays the Combination Index as calculated via CompuSyn Software, wherein the value 1 shows additivity, values smaller than 1 show supraadditivity and values above 1 show subadditivity or antagonism.

Shown are all dose combinations of MP1032 and dexamethasone as investigated, except for 3 values - for a better graphical overview only values up to a CI of 2 are depicted. All other CI values are below 1 and thus show a supraadditive effect.

### Fig. 3: FaCI fixed-ratio combinations - Example 1 - Experimental Part 2

The X coordinate displays the fractual effect (Fa), i.e. a value between 0 and 1 for the relative inhibition of IL-6 in LPS stimulated RAW264.7 cells, wherein 1 means a 100% inhibition and 0 means no inhibition. The Y coordinate displays the Combination Index as calculated via CompuSyn Software, wherein the value 1 shows additivity, values smaller than 1 show supraadditivity and values above 1 show subadditivity or antagonism. Shown are CI values calculated for various fractual effects including an estimated standard deviation.

Shown are calculations for fixed dose combinations of dexamethasone and MP1032 as follows: A 1: 10, B 1:40, C 1: 100 and D 1:200

## Claims

1. 5-amino-2,3-dihydro-1,4-phthalazinedione or one of its pharmaceutically acceptable salts for use as glucocorticoid-sparing agent allowing a significant reduction of the glucocorticoid dose compared to the use of the glucocorticoid alone in the prophylaxis or treatment of conditions treated with glucocorticoids.

2. 5-amino-2,3-dihydro-1,4-phthalazinedione or one of its pharmaceutically acceptable salts for use as glucocorticoid-sparing agent according to claim 1 **characterized in that** 5-amino-2,3-dihydro-1,4-phthalazinedione sodium salt is used.

3. 5-amino-2,3-dihydro-1,4-phthalazinedione or one of its pharmaceutically acceptable salts for use as glucocorticoid-sparing agent according to claim 1 or 2, wherein unwanted glucocorticoid effects are reduced or avoided.

4. Pharmaceutical combination comprising 5-amino-2,3-dihydro-1,4-phthalazinedione or one of its pharmaceutically acceptable salts and at least one glucocorticoid for use as defined in any one of claims 1 - 3 in the prophylaxis or treatment of conditions or diseases treated with glucocorticoids.

5. Pharmaceutical combination for use according to claim 4 wherein the glucocorticoid is selected from a group comprising glucocorticoids, flumethasone, triamcinolone acetonide, betamethasone, deflazacort, dexamethasone, beclomethasone, betamethasone valerate, betamethasone dipropionate, budesonide, beclomethasone dipropionate, isoflupredone, fluocinonide, fluocinolone, prednisone, prednisolone, methylprednisolone, halcinonide, desonide, deltasone, triamcinolone, triamcinolone acetonide, tixocortol pivalate, mometasone, amcinonide, fluocortolone, halometasone, alclometasone dipropionate, prednicarbate, clobetasone-17-butyrate, clobetasol-17-propionate, fluocortolone caproate, fluocortolone pivalate, fluprednidene acetate, ciclesonide, flunisolide, fluticasone furoate, fluticasone propionate, cortisol, hydrocortisone, hydrocortisone acetate, hydrocortisone-17-valerate, hydrocortisone-17-butyrate, hydrocortisone-17-aceponate, hydrocortisone-17-buteprate, cortisone and cortisone acetate.

6. Pharmaceutical combination for use according to claim 4 or 5, wherein the condition or disease is selected from a group comprising autoimmune disorders, allergies, cancers, adverse reactions associated to cancer therapies, muscular dystrophies, severe infectious diseases, systemic inflammatory diseases as well as inflammatory diseases of skin, respiratory system, gastrointestinal tract, urogenital system, cardiovascular system, musculoskeletal system, nervous system and sensory organs, and further conditions, such as cerebral edema, shock; sarcoidosis, hypercalcemia, adrenal insufficiency, adrenogenital syndrome, and acute mountain sickness; and to prevent transplant rejection.

7. Pharmaceutical combination for use according to any of claims 4 to 6, wherein the unwanted glucocorticoid effects to be reduced or avoided are selected from a group comprising hypertonia, weight gain, obesity, truncal obesity, corticosteroid-induced lipodystrophy, edema, puffiness of the face, potassium loss, muscle weakness, headache, facial hair growth in women, thinning of the skin, easy bruising, slow wound healing, glaucoma, cataracts, stomach and duodenum ulcers, acne, irregular menstrual cycle, steroid induced diabetes, loss of control in existing diabetes, osteoporosis, adrenal joint necrosis, psychiatric disturbances, psychotic behavior, growth retardation in children, convulsions, increased rate of infections, exacerbation of opportunistic infections, reduced effectiveness of antibiotics and vaccines, and Cushing's syndrome.

8. Pharmaceutical combination for use according to any of claims 4 to 7, wherein the glucocorticoid exhibits additionally mineralocorticoid potency.

9. Pharmaceutical combination for use according to any of claims 4 to 8, wherein 5-amino-2,3-dihydro-1,4-phthalazinedione or one of its pharmaceutically acceptable salts is either administered as add-on to the at least one glucocorticoid or within the same pharmaceutical composition with the at least one glucocorticoid.

10. Pharmaceutical composition for use as defined in claim 9, wherein the administration mode for the composition is selected from intravenous, oral, sublingual, rectal, topical and dermal administration.

11. Pharmaceutical composition for use according to claim 10 for oral application, wherein a dosage form is selected from tablets, soft gelatin capsules, hard gelatin capsules, sugar-coated tablets or pills; powders or granulates; juices, syrups, drops, teas, solutions or suspensions in aqueous or non-aqueous liquids; edible foams or mousses; or in oil-in-water or water-in-oil in emulsions.

12. Pharmaceutical composition for use according to claim 10 for inhalatory application,

13. Pharmaceutical composition for use according to claim 10 for topical application, wherein a dosage form is selected from creams, emulsions, lotions, gels, hydrogels, pastes, powders, ointments, liniment, films, liposomes, dermal patches, transdermal patches, transdermal sprays or suspensions.

## Patentansprüche

1. 5-Amino-2,3-dihydro-1,4-phthalazindion oder eines seiner pharmazeutisch verträglichen Salze zur Verwendung als Glucocorticoid-sparendes Mittel, das eine signifikante Verringerung der Glucocorticoid-Dosis in Hinsicht auf die Verwendung des Glucocorticoids allein in der Prophylaxe oder der Behandlung von Zuständen erlaubt, die mit Glucocorticoiden behandelt werden.

2. 5-Amino-2,3-dihydro-1,4-phthalazindion oder eines seiner pharmazeutisch verträglichen Salze zur Verwendung als Glucocorticoid-sparendes Mittel gemäß Anspruch 1, **gekennzeichnet durch** die Verwendung des Natriumsalzes von 5-Amino-2,3-dihydro-1,4-phthalazindion.

3. 5-Amino-2,3-dihydro-1,4-phthalazindion oder eines seiner pharmazeutisch verträglichen Salze zur Verwendung als Glucocorticoid-sparendes Mittel gemäß Anspruch 1 oder 2, wobei die unerwünschten Effekte der Glucocorticoide vermindert oder vermieden werden.

4. Pharmazeutische Kombination, umfassend 5-Amino-2,3-dihydro-1,4-phthalazindion oder eines seiner pharmazeutisch verträglichen Salze und mindestens ein Glucocorticoid zur Verwendung wie in den Ansprüchen 1 bis 3 definiert in der Prophylaxe oder der Behandlung von Zuständen oder Krankheiten, die mit Glucocorticoiden behandelt werden.

5. Pharmazeutische Kombination zur Verwendung gemäß Anspruch 4, wobei das Glucocorticoid aus einer Gruppe ausgewählt wird, die Glucocorticoide, Flumetason, Triamcinolon Acetonid, Betamethason, Deflazacort, Dexamethason, Beclometason Valerat, Betamethason Valerat, Betamethason Dipropionat, Budesonid, Beclometason Dipropionat, Isoflupredon, Fluocinonid, Fluocinolon, Prednison, Prednisolon, Methylprednisolon, Halcinonid, Desonid, Deltason, Triamcinolon Acetonid, Tixocortol Pivalat, Mometason, Amcinonid, Fluocortolon, Halometason, Alclomethason Dipropionat, Prednicarbat, Clobetason-16-Butyrat, Clobetasol-17-Propionat, Fluocortolon Caproat, Fluocortolon Pivalat, Flupredniden Acetat, Ciclesonid, Flunisolid, Fluticason Furoat, Fluticason Propionat, Cortisol, Hydrocortison, Hydrocortison Acetat, Hydrocortison-17-Valerat, Hydrocortison-17-Aceponat, Hydrocortison-17-Buteprat, Cortison und Cortison Acetat umfasst.

6. Pharmazeutische Kombination zur Verwendung gemäß Anspruch 4 oder 5, wobei der Zustand oder die Krankheit aus einer Gruppe gewählt wird, die AutoimmunErkrankungen, Allergien, Tumore, mit antitumoralen Therapien verbundene unerwünschte Reaktionen, Muskeldystrophien, schwere Infektionskrankheiten, systemische entzündliche Erkrankungen sowie entzündliche Krankheiten der Haut, des Atmungssystems, des Gastrointestinaltrakts, des Urogenitalsystems, des muskuloskelettalen Systems, des Nervensystems und der Sinnesorgane, sowie weitere Zustände wie Hirnödem, Schock, Sarkoidose, Hyperkalzämie, Nebenniereninsuffizienz, adrenogenitales Syndrom und Höhenkrankheit, und zur Prophylaxe einer Transplantatabstoßung umfasst.

7. Pharmazeutische Kombination zur Verwendung gemäß einem der Ansprüche 4 bis 6, wobei die zu vermindernden oder zu vermeidenden unerwünschten Effekte der Glucocorticoide aus seiner Gruppe ausgewählt werden, die Bluthochdruck, Gewichtszunahme, Fettleibigkeit, Fettleibigkeit des Rumpfes, durch Corticosteroide induzierte Lipodystrophie, Ödem, Schwellungen des Gesichts, Kaliumverlust, Muskelschwäche, Kopfschmerzen, Gesichtsbehaarung bei Frauen, Verdünnung der Haut, leicht induzierte Blutergüsse, langsame Vernarbung von Wunden, Glaukom, Katarakt, Geschwüre des Magens und des Duodenums, Akne, irregulärer Menstruationszyklus, durch Steroide induzierte Diabetes, Kontrollverlust eines vorexistierenden Diabetes, Osteoporose, Nekrose der Nebennierengelenke, psychiatrische Störungen, psychotisches Verhalten, Wachstumsverzögerung bei Kindern, Krämpfe, Steigerung der Infektionsrate, Verschlimmerung opportunistischer Infektionen, Wirkverringerung von Antibiotika und Impfstoffen und Cushing-Syndrom umfasst.

8. Pharmazeutische Kombination zur Verwendung gemäß einem der Ansprüche 4 bis 7, wobei das Glucocorticoid zudem eine mineralokortikoide Wirkung aufweist.

9. Pharmazeutische Kombination zur Verwendung gemäß einem der Ansprüche 4 bis 8, wobei 5-Amino-2,3-dihydro-1,4-phthalazindion oder eines seiner pharmazeutisch verträglichen Salze entweder als Add-On zu einem Glucocorticoid oder in derselben pharmazeutischen Zusammensetzung des mindestens einen Glucocorticoids verabreicht wird.

10. Pharmazeutische Zusammensetzung zur Verwendung wie im Anspruch 9 definiert, wobei die Administrationsroute der Zusammensetzung aus intravenöser, oraler, sublingualer, rektaler, topischer und dermaler Verabreichung ausgewählt wird.

11. Pharmazeutische Zusammensetzung zur Verwendung gemäß Anspruch 10 zur oralen Verabreichung, wobei die Darreichungsform aus Tabletten, Weichgelatinekapseln, Hartgelatinekapsel, mit Zucker überzogenen Tabletten oder Pillen, Pulvern oder Granulaten, Säften, Sirupe, Tropfen, Tees, in wässrigen oder nicht-wässrigen Lösungen oder Suspensionen, essbaren Schäumen oder Mousses oder Öl-in-Wasser-oder Wasser-in-Öl-Emulsionen ausgewählt wird.

12. Pharmazeutische Zusammensetzung zur Verwendung gemäß Anspruch 10 zur inhalativen Verabreichung.

13. Pharmazeutische Zusammensetzung zur Verwendung gemäß Anspruch 10 zur topischen Verabreichung, wobei die Darreichungsform aus Cremes, Emulsionen, Lotionen, Gelen, Hydrogelen, Pasten, Pulvern, Pomaden, Liniment, Filmen, Liposomen, Hautpflastern, transdermalen Pflastern, transdermalen Sprays oder Suspensionen ausgewählt wird.

## Revendications

1. 5-amino-2,3-dihydro-1,4-phtalazinedione ou l'un de ses sels pharmaceutiquement acceptables pour utilisation en tant qu'agent d'épargne glucocorticoïde permettant une réduction significative de la dose de glucocorticoïde par rapport à l'utilisation du glucocorticoïde seul dans la prophylaxie ou le traitement d'affections traitées par des glucocorticoïdes.

2. 5-amino-2,3-dihydro-1,4-phthalazinedione ou l'un de ses sels pharmaceutiquement acceptables pour utilisation en tant qu'agent d'épargne glucocorticoïde selon la revendication 1, **caractérisé par** l'utilisation du sel de sodium de 5-amino-2,3-dihydro-1,4-phthalazinedione.

3. 5-amino-2,3-dihydro-1,4-phthalazinedione ou l'un de ses sels pharmaceutiquement acceptables pour utilisation en tant qu'agent d'épargne glucocorticoïde selon la revendication 1 ou 2, dans lequel les effets glucocorticoïdes indésirables sont réduits ou évités.

4. Combinaison pharmaceutique comprenant 5-amino-2,3-dihydro-1,4-phtalazinedione ou l'un de ses sels pharmaceutiquement acceptables et au moins un glucocorticoïde pour l'utilisation telle que définie dans l'une des revendications 1 à 3 dans la prophylaxie ou le traitement de conditions ou de maladies traitées avec des glucocorticoïdes.

5. Combinaison pharmaceutique pour l'utilisation selon la revendication 4, dans laquelle le glucocorticoïde est choisi dans un groupe comprenant les glucocorticoïdes, flumétasone, acétonide de triamcinolone, bétaméthasone, deflazacort, dexaméthasone, valérate de béclométasone, valérate de bétaméthasone, dipropionate de bétaméthasone, budésonide, dipropionate de béclométasone, isoflupredone, fluocinonide, fluocinolone, prednisone, prednisolone, méthylprednisolone, halcinonide, désonide, deltasone, triamcinolone, acétonide de triamcinolone, pivalate de tixocortol, mométasone, amcinonide, fluocortolone, halométasone, dipropionate d'alclométasone, prednicarbate, 17-butyrate de clobétasone, 17-propionate de clobétasol, caproate de fluocortolone, pivalate de fluocortolone, acétate de fluprednidène, ciclésonide, flunisolide, furoate de fluticasone, propionate de fluticasone, cortisol, hydrocortisone, acétate d'hydrocortisone, 17-valérate d'hydrocortisone, 17-butyrate d'hydrocortisone, 17-acéponate d'hydrocortisone, 17-buteprate d'hydrocortisone, cortisone et acétate de cortisone.

6. Combinaison pharmaceutique pour l'utilisation selon la revendication 4 ou 5, dans laquelle l'affection ou la maladie est choisie dans un groupe comprenant les troubles auto-immuns, les allergies, les cancers, les réactions indésirables associées aux thérapies anticancéreuses, les dystrophies musculaires, les maladies infectieuses graves, les maladies inflammatoires systémiques ainsi que les maladies inflammatoires de la peau, du système respiratoire, du tractus gastro-intestinal, du système urogénital, du système cardiovasculaire, du système musculo-squelettique, du système nerveux et des organes sensoriels, et d'autres affections, telles que l'œdème cérébral, le choc, la sarcoïdose, l'hypercalcémie, l'insuffisance surrénalienne, le syndrome adrénogénital et le mal aigu des montagnes ; et pour prévenir le rejet de greffe.

7. Combinaison pharmaceutique pour l'utilisation selon l'une quelconque des revendications 4 à 6, dans laquelle les effets indésirables des glucocorticoïdes à réduire ou à éviter sont choisis dans un groupe comprenant l'hypertonie, la prise de poids, l'obésité, l'obésité tronculaire, la lipodystrophie induite par les corticostéroïdes, l'œdème, la bouffissure du visage, la perte de potassium, la faiblesse musculaire, les maux de tête, la pilosité faciale chez les femmes, l'amincissement de la peau, les ecchymoses faciles, la lenteur de la cicatrisation des plaies, glaucome, cataracte, ulcères de l'estomac et du duodénum, acné, cycle menstruel irrégulier, diabète induit par les stéroïdes, perte de contrôle du diabète existant, ostéoporose, nécrose des articulations surrénales, troubles psychiatriques, comportement psychotique, retard de croissance chez les enfants, convulsions, augmentation du taux d'infections, exacerbation des infections opportunistes, réduction de l'efficacité des antibiotiques et des vaccins, et syndrome de Cushing.

8. Combinaison pharmaceutique pour l'utilisation selon l'une quelconque des revendications 4 à 7, dans laquelle le glucocorticoïde montre en outre un pouvoir minéralocorticoïde.

9. Combinaison pharmaceutique pour l'utilisation selon l'une quelconque des revendications 4 à 8, dans laquelle 5-amino-2,3-dihydro-1,4-phtalazinedione ou l'un de ses sels pharmaceutiquement acceptables est administrée soit en complément d'au moins un glucocorticoïde, soit dans la même composition pharmaceutique que l'au moins un glucocorticoïde.

10. Composition pharmaceutique pour l'utilisation telle que définie dans la revendication 9, dans laquelle le mode d'administration de la composition est choisi parmi l'administration intraveineuse, orale, sublinguale, rectale, topique et dermique.

11. Composition pharmaceutique pour l'utilisation selon la revendication 10 pour application orale, dans laquelle la forme de dosage est choisie parmi les comprimés, les capsules de gélatine molle, les capsules de gélatine dure, les comprimés ou pilules enrobés de sucre, les poudres ou granulés, les jus, les sirops, les gouttes, les thés, les solutions ou suspensions dans des liquides aqueux ou non aqueux, les moussages ou mousses comestibles ou les émulsions huile-dans-eau ou eau-dans-huile.

12. Composition pharmaceutique pour l'utilisation selon la revendication 10 pour application par inhalation.

13. Composition pharmaceutique pour l'utilisation selon la revendication 10 pour application topique, dans laquelle la forme de dosage est choisie parmi les crèmes, les émulsions, les lotions, les gels, les hydrogels, les pâtes, les poudres, les pommades, le liniment, les films, les liposomes, les patchs dermiques, les patchs transdermiques, les sprays transdermiques ou les suspensions.
